# EUROPEAN PATENT SPECIFICATION

(11) **EP 1 395 648 B1**
(45) Date of publication and mention of the grant of the patent: **22.04.2009**
(21) Application number: 02741528.0
(22) Date of filing: 11.06.2002
(51) Int. Cl.: C12N 1/00, C07K 14/335, C07K 19/00, C12N 15/31, C12N 15/63

(54) **METHODS FOR BINDING ACMA-TYPE PROTEIN ANCHOR FUSIONS TO CELL-WALL MATERIAL OF MICRO-ORGANISMS**
VERFAHREN ZUR BINDUNG VON ACMA-TYP PROTEIN ANKER FUSIONEN AN MIKROORGANISMEN ZELLWANDMATERIALIEN
TECHNIQUE AMELIOREE PERMETTANT DE FIXER DES FUSIONS D'ANCRAGE DE PROTEINES DE TYPE ACMA A LA PAROI CELLULAIRE DE MICRO-ORGANISMES

(30) Priority: 11.06.2001 EP 01202239
(43) Date of publication of application: 10.03.2004
(73) Proprietor: Applied NanoSystems B.V., 9700 AC Groningen (NL)
(72) Inventor: LEENHOUTS, Cornelis, Johannes, NL-9753 KX Haren (NL); RAMASAMY, Ranjan, Colombo 05 (LK); STEEN, Anton, NL-9734 BR Groningen (NL); KOK, Jan, NL-9714 HL Groningen (NL); BUIST, Girbe, NL-9611 BN Sappemeer (NL); KUIPERS, Oscar, Paul, NL-9728 XG Groningen (NL)
(74) Representative: Prins, Adrianus Willem
(86) International application number: PCT/NL2002/000383
(87) International publication number: WO 2002/101026

(56) References cited:
- EP-A- 0 545 352
- WO-A-99/25836
- W.C. BROWN ET AL.: "Comparison of various procedures for removing proteins and nucleic acids from cell walls of Bacillus subtilis" PREPARATIVE BIOCHEMISTRY, vol. 6, no. 6, 1976, pages 479-488, XP001031191
- RECEP CIBIK ET AL.: "Identification of Mur, an atypical peptidoglycan hydrolase derived from Leuconostoc citreum" APPLIED AND ENVIRONMENTAL MICROBIOLOGY, vol. 67, no. 2, February 2001 (2001-02), pages 858-864, XP002187256

## Description

Heterologous surface display of proteins (Stahl and Uhlen, TIBTECH May 1997, 15, 185-192) on recombinant micro-organisms via the targeting and anchoring of heterologous proteins to the outer surface or cell-wall of host cells such as yeast, fungi, mammalian and plant cells, and bacteria has been possible for several years. Display of heterologous proteins at the surface of these cells has taken many forms, varying from the expression of reactive groups such as antigenic determinants, heterologous enzymes, (single-chain) antibodies, polyhistidyl tags, peptides, and other compounds. Heterologous surface display has been applied as a tool for applied and fundamental research in microbiology, molecular biology, vaccinology and biotechnology. Yet another application of bacterial surface display has been the development of live-bacterial-vaccine delivery systems. The cell-surface display of heterologous antigenic determinants has been considered advantageous for the induction of antigen-specific immune responses when using live recombinant cells for immunisation. Another application has been the use of bacterial surface display in generating whole-cell bioadsorbents or biofilters for environmental purposes, microbiocatalysts, and diagnostic tools.

In general, one has used chimeric proteins consisting of an anchoring or targeting part specific and selective for the recombinant organism used and has combined this part with a part comprising a reactive group as described above. A well known anchoring part for example comprise the so-called LPXTG box, that binds covalently to a *Staphylococcus* bacterial surface, i.e. in the form of a fully integrated membrane protein. In this way, chimeric proteins are composed of at least two (poly)peptides of different genetic origin joined by a normal peptide bond. For example, in patent application WO 94/18330 relating to the isolation of compounds from complex mixtures and the preparation of immobilised ligands (bioadsorbents), a method has been claimed for obtaining such a ligand which comprises anchoring a binding protein in or at the exterior of the cell wall of a recombinant cell. Said binding protein is essentially a chimeric protein produced by said recombinant cell, and is composed of an N-terminal part, derived from for example an antibody, that is capable of binding to a specific compound joined with a C-terminal anchoring part, derived from an anchoring protein purposely selected for being functional in the specific cell chosen. In WO 97/08553 provides a method for the targeting of proteins selectively to the cell wall of *Staphylococcus spp* only, using as anchoring proteins long stretches of at least 80-90 amino acid long amino acid cell wall-targeting signals derived from the lysostaphin gene or amidase gene of *Staphylococcus* which encode for proteins that selectively bind to *Staphylococcus* cell wall components.

Vaccine delivery or immunisation via attenuated bacterial vector strains expressing distinct antigenic determinants against a wide variety of diseases is now commonly being developed. Recently, mucosal (for example nasal or oral) vaccination using such vectors has received a great deal of attention. For example, both systemic and mucosal antibody responses against an antigenic determinant of the hornet venom were detected in mice orally colonised with a genetically engineered human oral commensal *Streptococcus gordonii* expressing said antigenic determinant on its surface (Medaglini et al., PNAS 1995, 2; 6868-6872). Also, a protective immune response could be elicited by oral delivery of a recombinant bacterial vaccine wherein tetanus toxin fragment C was expressed constitutively in *Lactococcus lactis* (Robinson et al., Nature Biotechnology 1997, 15; 653-657). Especially mucosal immunisation as a means of inducing IgG and secretory IgA antibodies directed against specific pathogens of mucosal surfaces is considered an effective route of vaccination. Immunogens expressed by bacterial vectors are presented in particulate form to the antigen-presenting cells (for example M-cells) of the immune system and should therefore be less likely to induce tolerance than soluble antigens. In addition, the existence of a common mucosal immune system permits immunisation on one specific mucosal surface to induce secretion of antigen-specific IgA, and other specific immune responses at distant mucosal sites. A drawback to this approach is the potential of the bacterial strain to cause inflammation and disease in itself, potentially leading to fever and bacteraemia. An alternative approach avoids the use of attenuated bacterial strains that may become pathogenic themselves by choosing recombinant commensal bacteria as vaccine carriers, such as *Streptococcus spp.* and *Lactococcus spp*.

However, a potential problem with such recombinant organisms is that they may colonise the mucosal surfaces, thereby generating a long term exposure to the target antigens expressed and released by these recombinant micro-organisms. Such long term exposure can cause immune tolerance. In addition, the mere fact alone that such organisms are genetically modified and contain recombinant nucleic acid is meeting considerable opposition from the (lay) public as a whole, stemming from a low level of general acceptance for products containing recombinant DNA or RNA. Similar objections exist against the use of (even attenuated) strains of a pathogenic nature or against proteins or parts of proteins derived from pathogenic strains. However, as explained above, present techniques of heterologous surface display of proteins in general entail the use of anchoring or targeting proteins that are specific and selective for a limited set of micro-organisms which in general are of recombinant or pathogenic nature, thereby greatly restricting their potential applications.

The protein anchor of *L. lactis*, AcmA (cA), its homologs and functional derivatives (WO99/25836) binds in a non-covalent manner to a wide variety of Gram-positive bacteria. Binding also occurs to isolated cell wall material. The binding ligand in these cell walls is currently unknown. Use of a gram-positive, food-grade bacterium, such as *Lactococcus lactis*, offers significant advantages over use of other bacteria, such as *Salmonella*, as a vaccine delivery vehicle. *L. lactis* does not replicate in or invade human tissues and reportedly possesses low intrinsic immunity (Norton et aL 1994). *L. lactis* expressing tetanus toxin fragment C has been shown to induce antibodies after mucosal delivery that protect mice against a lethal challenge with tetanus toxin even if the carrier bacteria where killed prior to administration (Robinson et al. 1997). Nevertheless, these bacteria still contain recombinant DNA that will be spread into the environment, especially when used in wide-scale oral-immunization programmes. This uncontrollable shedding of recombinant DNA into the environment may have the risk of uptake of genes by other bacteria or other (micro)organisms.

The invention discloses a method for improving binding of a proteinaceous substance to cell-wall material of a Gram-positive bacterium, said substance comprising at least one repeat, but preferably two or three repeat sequences of an AcmA cell wall binding domain or homolog or functional derivative thereof, said method comprising treating said cell-wall material with a solution capable of removing a cell-wall component such as a protein, (lipo)teichoic acid or carbohydrate from said cell-wall-material and contacting said substance with said treated cell-wall material. Improved binding is obtained in said method by treating said cell-wall material with a solution capable of removing a cell-wall component, optionally followed by storage of the thus obtained cell-wall material until it is provided with the desired (fusion) protein by providing said protein with AcmA cell wall binding domain or homolog or functional derivative thereof and contacting the cell-wall material with the protein. By applying a method according to the invention, cell-wall material is obtained with improved capacity for binding of a proteinaceous substance comprising an AcmA cell wall binding domain or homolog or functional derivative thereof.

As said, the invention provides a method to remove components from a bacterial call-wall that comprises the treatment of whole cells with a solution capable of removing a cell-wall component such as a protein, (lipo)teichoic acid or carbohydrate from said cell-wall material Cell-wall material obtained by the invention essentially yields cell-wall material that exists for at least 20%, better 30%, best 40% or preferably 50% of relatively emptied but intact cell envelopes essentially comprised of inert spherical microparticles, further referred to as bacterial "ghosts". Ghosts reflect the size and shape of the bacterium from which they are obtained.

The invention discloses a method for obtaining cell-wall material of a Gram-positive bacterium with improved capacity for binding with a proteinaceous substance comprising an AcmA cell wall binding domain or homolog or functional derivative thereof, said method comprising treating said cell-wall material with a solution capable of removing a cell-wall component such as a protein, (lipo)teichoic acid or carbohydrate from said cell-wall material
wherein said cell-wall material essentially comprises spherical peptidoglycan microparticles herein referred to as ghosts. Provided is a method according to claim 1, and spherical peptidoglycan microparticles obtainable thereby.

Methods to extract bacterial cell-wall material with a solution were described in for example, EP 0 545 352 A and Brown et al. (Prep. Biochem. 6:479 (1976) present a method to obtain purified soluble peptidoglycan from bacteria by exposure to TCA. These publications describe procedures in which cells are first mechanically disrupted and the resulting cell fragments are subsequently treated with TCA to extract peptidoglycans from the cell-wall. However, cited methods provide a peptidoglycan preparation and a lysed, randomly fragmented cell-wall preparation from which cell-wall components have been removed. It is clear that said methods do not yield ghosts. Furthermore, said methods in essence do not allow targeting with a proteinaceous substance comprising an AcmA cell-wall binding domain or homolog or functional homolog thereof. In contrast, the method in the invention is preferably aimed at yielding ghosts from which cell-wall components have been removed. However, the use of ghosts for display of proteinaceous substances has advantages over the use of disrupted cell-wall material. For example, binding of a proteinaceous substance to bacterial ghosts results in a higher packing density compared to binding of a substance to mechanically disrupted cell-wall material. A high density surface display of proteins is favourable for application in industrial processes. The invention provides a method not involving rupture of the cell-wall material.

The use of cell-wall material obtained by a method involving mechanical disruption methods suffers from several practical drawbacks. Because cells are broken completely, all intracellular materials are released and cell wall fragments must be separated from a complex mixture of proteins, nucleic acids, and other cellular components. Released nucleic acids may increase the viscosity of the solution and may complicate subsequent processing steps and especially chromatography. The cell debris, produced by mechanical lysis, often consists of small cell fragments. These are difficult to remove. Such problems are overcome when using ghosts. The uniform composition of a ghost preparation with respect to particle size and shape offers further advantages for subsequent purification and isolation steps. The invention provides cell-wall material obtainable by a method not involving rupture of the cell-wall wherein said cell-wall material comprises ghosts.

Bacterial ghosts are often preferable to the use of disrupted cell-wall bacteria for the surface display of immunogenic determinants. In contrast to the disrupture procedure, ghosts are inactivated through a process that preserves much of the bacteria's native spherical structure. Bacterial ghosts are thus better able to bind to and/or are more easily taken up by specific cells or tissues. The ability of bacterial ghosts to target macrophages or dendritic cells enhances their functional efficacy. Thus, the non-recombinant non-living ghost system provided by the invention is especially suited as vaccine delivery vehicle. The invention thus provides a method for obtaining ghosts with improved capacity for binding with a proteinaceous substance and with enhanced induction of cellular immune respons. The invention discloses a method for binding of a proteinaceous substance to cell-wall material of a Gram-positive bacterium, said substance comprising an AcmA cell wall binding domain or homologue or functional derivative thereof, said method comprising treating said cell-wall material with a solution capable of removing a cell-wall component such as a protein, (lipo)teichoic acid or carbohydrate from said cell-wall material and contacting said substance with said cell-wall material wherein said cell-wall material comprises ghosts which have been obtained by a method which does not involve rupture of the bacterial cell-wall. Provided is a method according to claim 16.

In a preferred embodiment, said solution has a pH that is lower than the calculated Pi value of said AcmA call wall binding domain or homolog or functional derivative thereof, in particular wherein said solution comprises an acid. Such an acid is preferably selected from the group of acetic acid (HAc) hydrochloric acid (HCl), sulphuric acid (H₂SO₄), trichloroacetic acid (TCA), trifluoroacetic acid (TFA), and monochloro acetic acid (MCA). The concentration of said acid in said solution is dependent on the desired pH value to be reached, which pH value is determined mostly by the appropriate calculated can for example be done by computer programmes such as DNA star or Clone Manager. When calculated pI is for example > 8 pH values of 6 to 4 suffice for effecting approriate binding; when pI value are lower, such as around 6, pH values of 3-4 are preferably selected With domains with calculated pI values ranging from 8 to 12, it for example suffices when said solution comprises 0.06 to 1.2 M TCA or comparable acid.

It is preferred to further effect the improvement of binding by heating said cell-wall material or ghosts in said solution, although, again, precise requirements for said heating need not be set. Heating for for example 5 - 25 minutes at around boiling temperature (100 °C) is in general more than enough to generate the desired cell-wall material with further improved binding capacity. Said material can than advantageously be washed and pelleted (e.g. by centrifugation) from said treatment solution and, if so desired, advantageously be stored (e.g. by freezing) or freeze-dried and stored until further use. Such cell-wall material essentially comprises spherical peptidoglycan microparticles that reflect the size and shape of the bacterium from which they are obtained.

In a preferred embodiment, such cell-wall material is derived from a *Lactococcus, a Lactobacillus, a Bacillus* or a *Mycobacterium spp*. Cell walls of Gram-positive bacteria consist of complex networks of peptidoglycan layers, proteins, (lipo)teichoic acids and other (modified) carbohydrates. In general, chemical treatment of cell wall material is used to remove cell wall components like proteins, (lipo)teichoic acids and carbohydrates to yeld purified peptidoglycan (Morata de Ambrosini et al. 1998). Sodium dodecyl sulphate (SDS) is commonly used to remove proteins. Trichloric acid (TCA) is known to remove specifically lipoteichoic acids and carbohydrates from cell wall isolates. Phenol, formamide and mixtures of chloroform and methanol are other examples of organic solvents that are used to enhance the purification of peptidoglycan.

Here we studied the effect of pretreatment of whole cells of gram-positive bacteria with these and other chemicals on binding technology that provides the possibility to give bacterial ghosts or cell-wall material derived from said bacteria new traits without introducing recombinant DNA into them.

In a preferred embodiment, the invention provides the use of said cell-wall material with improved binding capacity for AcmA-type anchors for the preparation of a composition, such as pharmaceutical composition, with a proteinaceous substance comprising an AcmA-type anchor. Reactive groups such as antigenic determinants, heterologous enzymes, (single-chain) antibodies, polyhistidyl tags, peptides, and other compounds can now easily be bound to the cell-wall material as provided herein by providing said groups with an AcmA -type anchor and contacting with the cell-wall material with improved binding capacity. Other reactive groups are, fluorescing protein, luciferase, binding protein or peptide, or another substance such as an antibiotic, hormone, non-peptide antigenic determinant, carbohydrate, fatty acid, aromatic substance and reporter molecule.

In another preferred embodiment, the invention provides the use of cell wall material as provided herein in generating bioadsorbents or biofilters for environmental purposes, microbiocatalysts, and diagnostic tools. For example the use of immobilized biocatalysts (enzymes or whole microbial cells) in the food, pharmaceutical and chemical industries has increased steadily during the past decade. Immobilized biocatalysts are generally more stable, easier to handle than their free counterparts, and most importantly is that they can be used repeatedly in industrial processes. At present, immobilization of enzymes often requires a chemical step linking the enzyme to an insoluble support. However, chemical treatments may influence the enzymes negatively. Alternatively, enzymes are immobilized by incorporation in gels with the obvious disadvantage that diffusion of the substrate into the gel is slowing down the process.

An alternative provided herein is the surface display of proteins on gram-positive cells or cell-wall material as provided by the invention for use in efficient large-scale immobilization of enzymatically active proteins. Immobilization of a fusion protein comprising α-amylase or β-lactamase fused to the AcmA-protein anchor domain has been demonstrated in *L. lactis*, herein. Addition of an AcmA-anchor fusion protein resulted in stable attachment of heterologous proteins to the surface of *L. lactis* and other gram-positive bacteria. Furthermore, acid pre-treatment of *L. lactis* cells and other gram-positive cells, described in the present invention, results in a high density surface display of heterologous proteins which is a prerequisite for application in industrial processes. Moreover, the carrier, e.g. gram-positive cells, can be obtained in high yield and is non-recombinant. Therefore, a method as provided herein can economically produce the immobilized enzyme and makes the AcmA-protein anchor useful approach for surface display of enzymes on gram-positive cells.

Another representative industrial application of an immobilized enzyme is the isomerization of glucose, during production of high-fructose corn syrup, catalyzed by glucose isomerase. This process can only be made economically feasible by immobilization of the enzyme. Increasing the stability of epoxide hydrolase in organic solvents by immobilization to microbial cells, or cell wall material, as provided herein, increases productivity of this enzyme. Immobilized enzymes can also be used for the treatment of waste water or industrial effluent. For example, the treatment of industrial effluent containing low value chemicals produced during synthesis of commodity chemicals epichlorohydrin and propylene oxide. Immobilized haloalkane dehalogenase is used to recycle these low value products into the manufacturing process.

The invention also discloses chimeric or hybrid AcmA-type anchors for the preparation of a composition according to the invention that have new binding properties. Based on the pI of the AcmA-type anchors they can be divided into two groups of hybrids (table 3): a large group with a pI higher than 8 but lower than 10 and a smaller group with a relatively low pI (<5). Preferred hybrid AcmA type anchors have at least one AcmA type domain with relative high calculated pI, and one with relative lower calculated pL The resulting anchor has an intermediate calculated pI, which is for example useful when release of the bound proteinaceous substance at higher pH is contemplated, for example when a composition comprising a cell-wall material according to the invention is provided with an chimeric anchor according to the invention. Such a composition may than be routed through the stomach, which has relative low pH and may more easily release its anchor bound reactive groups when the intestines are reached.

The invention thus discloses proteinaceous substance comprising an AcmA cell wall binding domain or homolog or functional derivative thereof wherein said domain is a hybrid of at least two different AcmA-type cell wall binding domains or homologs or functional derivatives thereof. Preferably, the invention provides a proteinaceous substance comprising an AcmA cell wall binding domain or homolog or functional derivative thereof wherein said domain is a hybrid of at least two different AcmA repeat sequences wherein said hybrid has a calculated pI lower than 10. For example, the invention provides a hybrid protein anchor composed of the A1 and A2 repeat sequences of AcmA and the D1 repeat sequence of AcmD. It is preferred that such a hybrid domain comprises at least one AcmA type domain with relative high calculated pI, and one with relative lower calculated pI. For a domain with relative high pI, it is preferred to select at least one domain that is derived from or functionally equivalent to the AcmA type domain of the lactococcal cell wall hydrolase AcmA, but many others with high pI are known, for example from table 3. For a domain with relative low pI, it is preferred to select at least one domain that is derived from or functionally equivalent to the AcmA type domain of the lactococcal cell wall hydrolase AcmD, but many others with relative low pI are known, for example from table 3. Disclosed is proteinaceous substance comprising a hybrid domain wherein at least two stretches of amino acids, each corresponding to a domain repeat sequence, are located adjacent to each other, possibly separated by one or more amino acid residues. Said stretches or repeats can be separated by a short distance, for example 3-6 to 10-15 amino acids apart, or by a medium distance 15-100 amino acids apart, or by longer distances (>100 amino acid residues apart).

The invention discloses a proteinaceous substance with a hybrid AcmA domain, which is additionally comprising a reactive group. For example, a proteinaceous substance comprises a reactive group such as an antigenic determinant, heterologous enzyme, (single-chain) antibody or fragment thereof, polyhistidyl tag, fluorescing protein, luciferase, binding protein or peptide, or another substance such as an antibiotic, hormone, non-peptide antigenic determinant, carbohydrate, fatty acid, aromatic substance, inorganic particle such as latex, or reporter molecule, and an AcmA cell wall binding domain or homolog or functional derivative thereof
wherein said domain is a hybrid of at least two different AcmA cell wall binding domains or homologs or functional derivatives thereof, (herein also called hybrid AcmA domain) useful in heterologous surface display which is both broadly reactive with cell wall components of a broad range of micro-organisms.

For example, said reactive group is a non-protein moiety, for example is selected from the group of antibiotics, hormones, aromatic substances, inorganic particles, and reporter molecules. Said substance is constructed by binding for example an antibiotic, such as penicillin or tetracycline, but various other antibiotics can be used, or a hormone, such as a steroid hormone, or any other compound to an binding domain provided by the invention. Such binding can be achieved by various techniques known in the art, and thereby can label or "flag" the binding domain- A preferred example is the binding of an binding domain to a reporter molecule such as fluorescent nanoparticles, FITC or HRPO, whereby tools are generated that can be used in diagnostic assays whereby micro-organisms having peptidoglycan are detected. Similarly, a binding domain with an antibiotic bound there can be used *in vivo* by for example parenteral administration into the bloodstream of humans or animals or *in vitro* to bind to such micro-organisms having peptidoglycan, thereby increasing the concentration of antibiotic around said organism, which then gets killed by the antibiotic action.

The invention discloses a substance wherein said reactive group is a protein moiety, for example selected from the group of antigenic determinants, enzymes, (single-chain) antibodies or fragments thereof, polyhistidyl tags, fluorescing proteins, binding proteins or peptides. For example, the invention provides a protein, which comprises as reactive group a protein or (poly)peptide. Also, the invention provides a nucleic acid molecule encoding a protein provided by the invention. Such a nucleic acid molecule (being single- or double stranded DNA, RNA or DNA/RNA) at least comprises nucleic acid sequences specifically encoding a hybrid binding domain, and may as well comprise nucleic acid sequences specifically encoding the reactive group polypeptide, but can additionally also comprise other nucleic acid sequences, which for example encode a signal peptide, or comprise for example promoter and/or regulatory nucleic acid sequences. The invention also discloses a vector comprising a nucleic acid molecule encoding a proteinaceous substance provided by the invention. The invention also discloses a vector comprising a nucleic acid molecule encoding a proteinaceous substance provided by the invention. Such a vector can for example be a plasmid, phage, or virus, and can now be constructed using a nucleic acid provided by the invention and routine skills of the art. Examples of such a vector can be found in the experimental part of the description, other examples can e.g. be a baculovirus vector, or comparable vector viruses through which a protein provided by the invention can be expressed or produced in (insect)cells. The invention also discloses a host cell or expression system comprising a nucleic acid molecule according to the invention or a vector according to the invention. Such a host cell expressing a protein is in it self provided by the invention as a micro-organism to which a protein provided by the invention is attached. Such a host cell or expression system can for example be a Gram-positive- or Gram-negative bacterium, or a yeast cell or insect cell or plant- or mammalian cell, or even a cell-free expression system such as a reticulocyte lysate, and can now be constructed or obtained using a nucleic acid or vector provided by the invention and routine skills of the art. Examples of such a host cell or expression system can be found in the experimental part of the description, other examples can be obtained using a nucleic acid or vector provided by the invention and routine skills of the art.

In a further preferred embodiment, the invention provides a pharmaceutical composition comprising said cell-wall material with improved binding capacity and an immunogen bound thereto, which is useful for vaccination purposes, a vaccine. In particular, the invention provides a vaccine to elicit immunity for pathogens, like malaria, that undergo stages in their life cycle where they are not in the blood but hide in cells.

Vaccines delivered to mucosal surfaces are sometimes preferable to injectible vaccines. They are easier and safer to administer. For mucosal vaccination, *L. lactis* derived cell-wall material may advantageously be used considering that this bacterium is of intestinal origin to which no distinct adverse immune reactions are generally to be expected. However, for application of a vaccine according to the invention *per injectionem*, it is useful to consider cell-wall material derived from a *Mycobacterium spp*, considering the beneficial adjuvant properties of Mycobacterial cell wall preparations, and provide it with a proteinaceous substance that carries the necessary immunogenic determinant(s) for use as a vaccine.

Especially when considering that by applying a method according to the invention the cell-wall material of the invention is likely at least partly depleted of unwanted immunogens that may elicit undesirable immune responses directed against bacterial cell-wall components, such a vaccine carries reduced risks of generating undesirable immune responses against cell-wall components.

### Figure legends

Fig. 1. Schematic map of plasmid pNG3041 that encodes the reporter protein MSA2::cA, which is secreted as a proprotein using the lactococcal PrtP signal-and prosequences (PrtP.sspro). Pnis: the nisin inducible promoter of the *nisA* gene. T: transcriptional terminator. CmR: chloramphenicol resistance gene. repC and repA: genes involved in the replication of the plasmid.
Fig. 2. Fluorescence microscopic images of bacterial cells with externally bound MSA2::cA. A. *Lb. curvatis, Lb. sake* and *L. lactis* cells that were not pretreated prior to binding. B. *L. lactis* cells that were TCA pretreated prior to binding. The light colored areas indicate the position were the reporter protein MSA2::cA binds. The difference between *L. lactis* cells that were not pretreated with TCA (in A) and those there were TCA pretreated is obvious (in B).

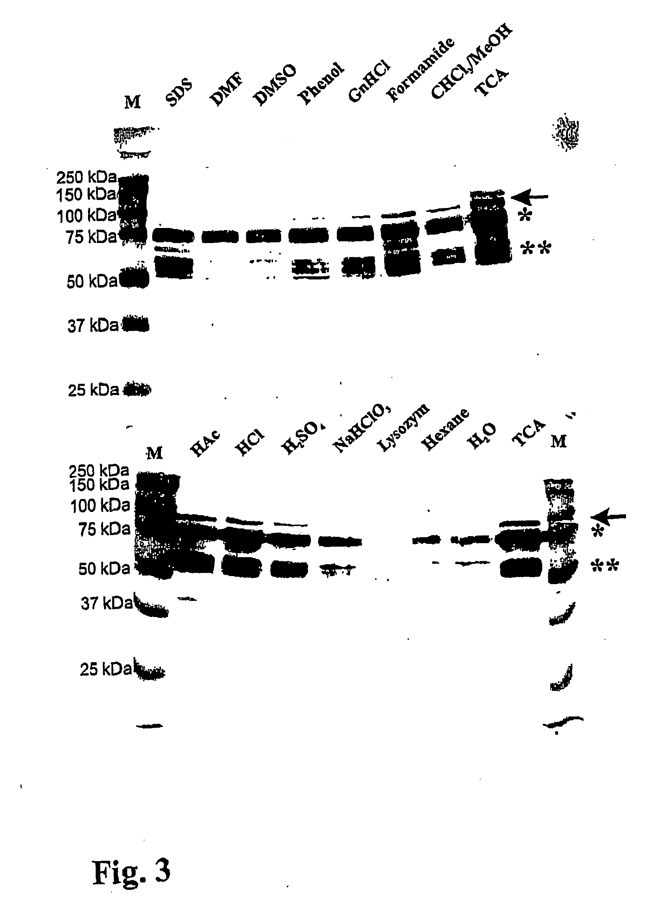

Fig. 3. Western blots of chemically pretreated *L. lactis* cells that were washed after the pretreatment and, subsequently, incubated with MSA2::cA to allow binding. Unbound MSA2::cA was removed by washing. The picture shows the MSA2::cA that had been bound to the chemical pretreated cells, detected using an antibody specific for MSA2. The different pretreatments are indicated above the lanes. MSA2::cA is produced by the producer cells as a proprotein: pro-MSA2::cA, some of this is present in the medium used for binding and this also binds (indicated by the arrow). A membrane bound protease, HtrA, of the producer cells cleaves off the pro-sequence resulting in mature MSA2::cA, which also binds to the pretreated cells (indicated by the asterisk). HtrA also cleaves off the repeats of the cA anchor. Since there are three repeats, MSA2 proteins of several sizes are present in the medium of the producer. As long as more than one repeat is present binding can still occur. The double asterisks points to MSA2::cA from which one or two repeats have been cleaved off. M: molecular weight marker (the molecular weights are indicated in the left margin). The two blots differ in signal intensity. As a reference they both contain the same TCA-pretreated samples. The difference in signal intensity is due to differences in stain developing time. It can clearly be seen that the TCA and other acid pretreatments have the most pronounced effect on the subsequent binding of MSA2::cA.
   The conclusions for all chemical pretreatments are summarized in Table 1.
Fig. 4. Coomassie stained SDS-PAGE gel with chemically pretreated *L. lactis* cells.
   Pretreatments:
   1. No-treatment
   2. HCl
   3. H₂SO₄
   4. HAc
   5. TFA
   6. TCA
   It is clear that treatment of the cells with HCl, H₂SO₄, TFA or TCA removes significant amounts of proteins from the cells.
Fig. 5. Western blot of *L. lactis* cells TCA pretreated with different TCA concentrations and bound externally with MSA2::cA. Arrow and asterisks: as in Fig. 3.
   Pretreatments:
   1. No TCA-treatment
   2. 1% TCA
   3. 5% TCA
   4. 10% TCA
   5. 20% TCA
   The Figure shows a clear increase in the binding of MSA2::cA with increasing amounts of TCA used in the pretreatment.
Fig. 6. Alignment of cA repeats with cD repeats. The amino acids that are in agreement with the consensus sequence as shown at the bottom of the Figure (defined in WO99/25836) are underlined. The asterisks indicate residues that are identical between the repeats compared.
Fig. 7. Binding of different anchor-fusion proteins to *L. Lactis* with and without TCA pretreatment. The principle is the same as in Fig. 3. Multiple bands in one lane are caused by the different processed forms of MSA2 fusions.
   1. Non-pretreated *L. lactis* + MSA2::cA
   2. Non-treated *L. lactis* + MSA2::cD
   3. Non-pretreated *L. lactis* + MSA2
   4. TCA-pretreated *L. lactis* + MSA2::cA
   5. TCA-pretreated *L. lactis* + MSA2::cD
   6. TCA-pretreated *L. lactis* + MSA2
   The effect of TCA pretreatment on the binding of MSA2::cA is evident (compare lanes 1 and 4). There seems a minor improvement for MSA2::cD and no improvement for MSA2 without anchor. That there is a signal for MSA2 without anchor means that MSA2 itself has a weak affinity for bacterial cell walls. However, MSA2::cD or MSA2 binding to the pretreated cells can not be detected using fluorescence - and electron microscopy (see text). This difference in results is most likely due to a difference in sensitivity of these techniques.

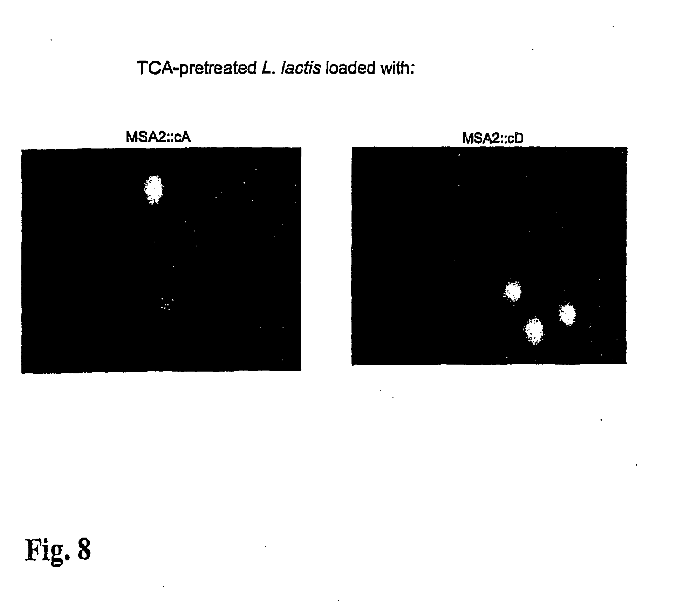

Fig. 8. Fluorescence microscopy image of TCA-pretreated *L. lactis* cells incubated with MSA2::cA or MSA2::cD. Light colored areas indicate the position were the reporter fusion protein bound. It is clear that binding only occurred with MSA2::cA and not with MSA2::cD.

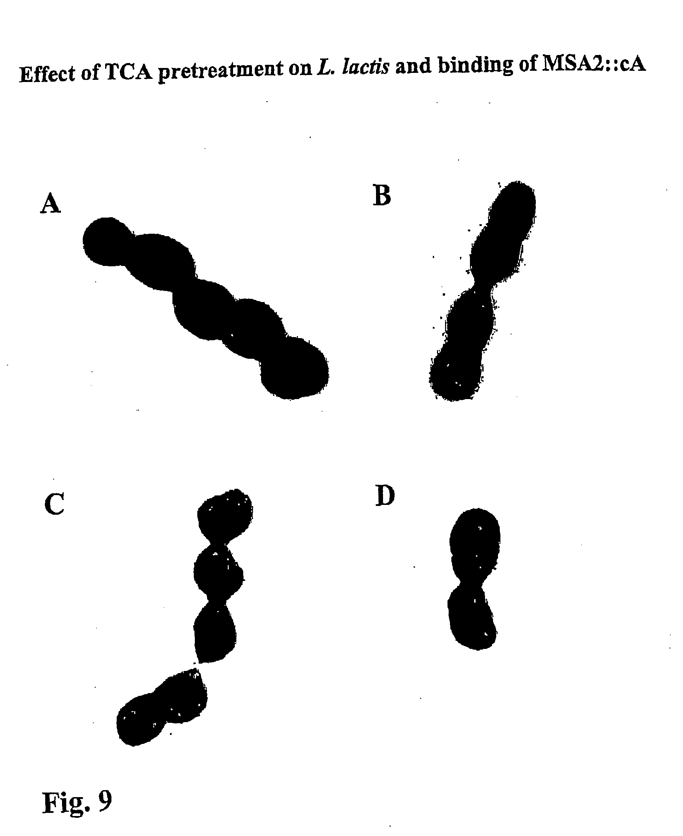

Fig. 9. Electron microscopy images of *L. lactis* cells incubated with different MSA2 constructs. The black dots represent the position of bound MSA2 (fusion) protein.
   A. Non-pretreated cells incubated with MSA2::cA.
   B. TCA-pretreated cells incubated with MSA2::cA.
   C. TCA-pretreated cells incubated with MSA2::cD.
   D. TCA-pretreated cells incubated with MSA2.
      Significant binding (black dots) is only visible in the TCA-pretreated cells incubated with MSA2::cA (B).

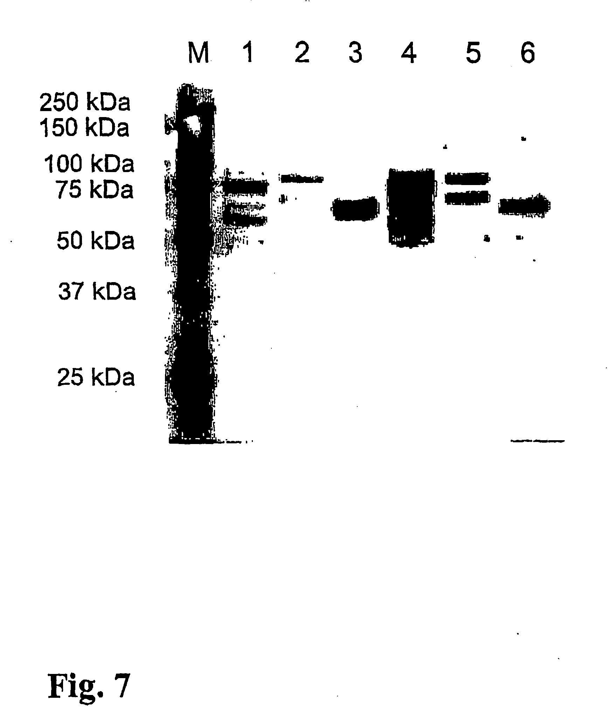

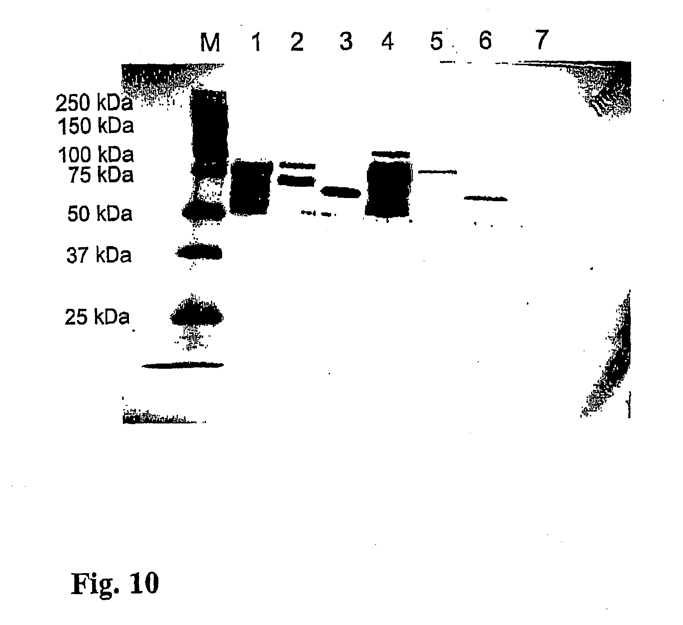

Fig. 10. Binding of different anchor-fusion proteins to *B. subtilis* with and without TCA pretreatment. The picture is a Western blot similar as in Figs. 3 and 7.
   1. Non-pretreated cells + MSA2::cA
   2. Non-pretreated cells + MSA2::cD
   3. Non-pretreated cells + MSA2
   4. TCA-pretreated cells + MSA2::cA
   5. TCA-pretreated cells + MSA2::cD
   6. TCA-pretreated cells + MSA2
   7. Non-pretreated *B. subtilis* (negative control)
   From this picture it is clear that TCA pre-treatment improves the binding of MSA2::cA (compare lanes 1 and 4) in a similar way as it does for *L. lactis*. For MSA2::cD and MSA2 without anchor only some background binding is observed.

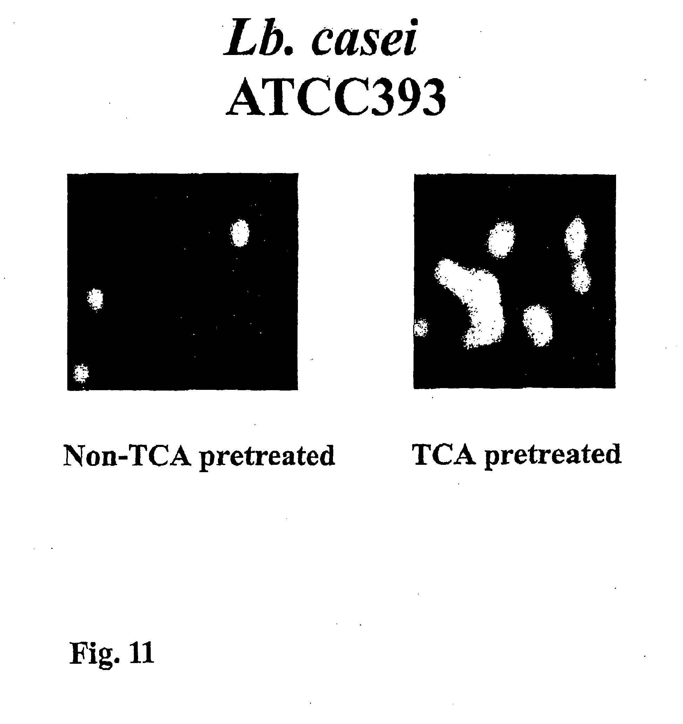

Fig. 11. Fluorescence microscopy image of MSA2::cA binding to *Lb. casei* with or without TCA pretreatment. The light colored areas represent bound MSA2::cA. It is evident that TCA pre-treatment improves binding of MSA2::cA also for *Lb. casei*.

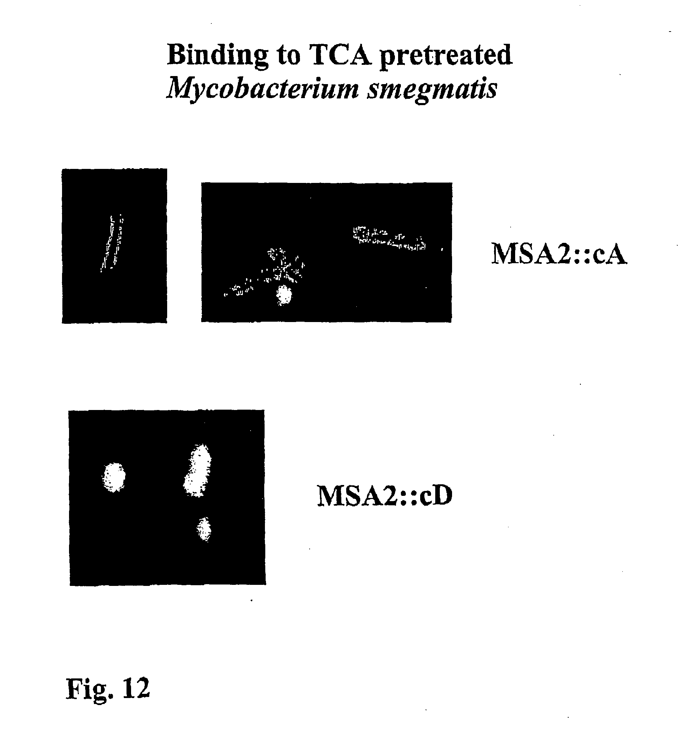

Fig. 12. Fluorescence microscopy image of MSA2::cA and MSA2::cD binding to M. *smegmatis* pretreated with TCA. The light colored areas represent bound MSA2 fusion protein. Clearly visible is that also in this case only MSA2::cA binds.

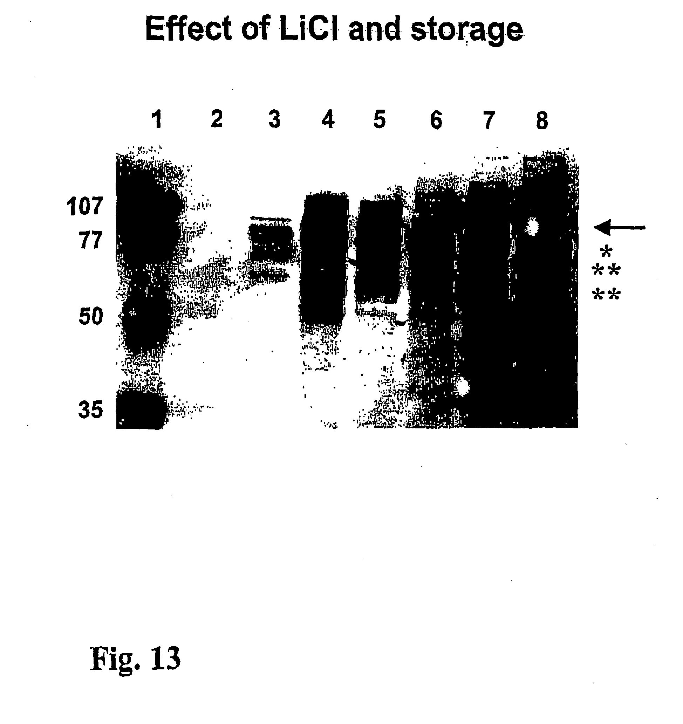

Fig. 13. Western blot of *L. lactis* cells with externally bound MSA2::cA treated with LiCl or stored under different conditions. The bands in the different lanes represent the amount of MSA2::cA that remained bound to the TCA pretreated cells. Arrow and asterisks as in Fig. 3.
   1. Marker
   2. Non-pretreated *L. lactis* not incubated with MSA2::cA.
   3. Non-pretreated *L. lactis* incubated with MSA2::cA.
   4. TCA-pretreated *L. lactis* incubated with MSA2::cA.
   5. TCA-pretreated *L. lactis* incubated with MSA2::cA, subsequently washed with 8M
      LiCl.
   6. TCA-pretreated *L. lactis* incubated with MSA2::cA, subsequently stored in water for 3 weeks at 4 C.
   7. TCA-pretreated *L. lactis* incubated with MSA2::cA, subsequently stored in 10%
      glycerol for 3 weeks at -80 C.
   8. TCA-pretreated *L. lactis* incubated with MSA2::cA, subsequently stored in water for three weeks at -80 C.
      This Figure shows again the beneficial effect of TCA pretreatment on the binding of MSA2::cA to *L. lactis* cells (compare lanes 3 and 4). Clearly visible is also that washing with 8 M LiCl and storage in water for 3 weeks at 4 °C has only minor effects on the bound MSA2::cA (compare lane 4 with 5 and 6). Storage at -80°C has no effect on the bound MSA2::cA (compare lane 4 with 7 and 8).

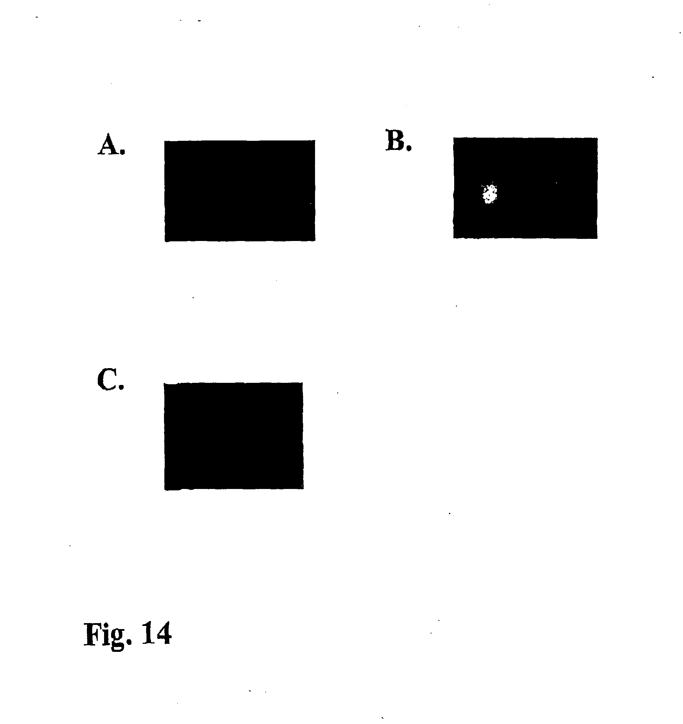

Fig. 14. Fluorescence microscopy image of (A) MSA2::cA and (B) MSA2::cP surface expression in the recombinant strains NZ9000(pNG3041) and NZ9000(pNG3043), respectively. (C) MSA2::cA binding to TCA-pretreated *L. lactis* cells. The light colored areas indicate the position of MSA2 fusion protein. The recombinant strain producing MSA2::cA has the protein on the surface only in some specific spots (A). The recombinant strain producing MSA2::cP has more on the surface organized in several areas (B) and the surface of the TCA-pretreated non-recombinant *L. lactis* with bound MSA2::cA is clearly completely covered with the protein (C).

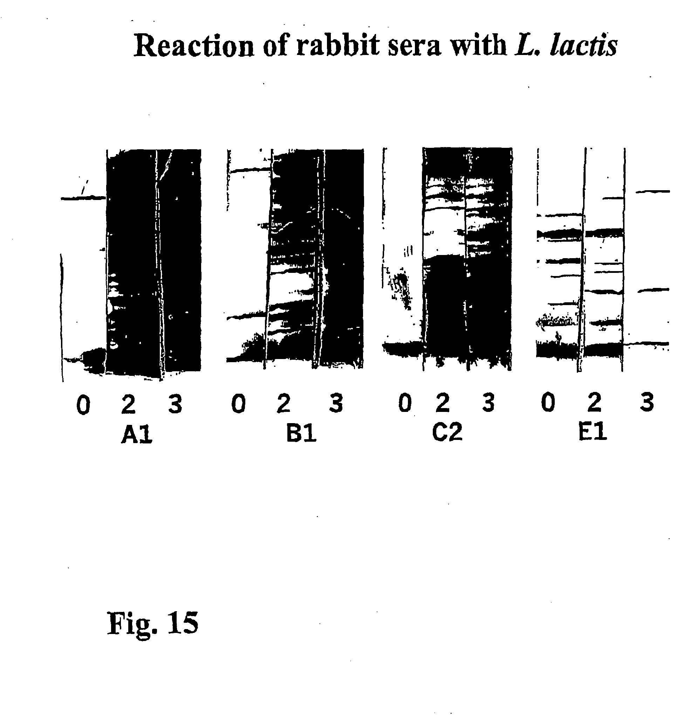

Fig. 15. Western blots of *L. lactis* total protein extracts reacted with rabbit immune serum. Serum dilution 1:100.
   0: preimmune serum. 2 and 3: serum after the second and third immunization, respectively.
   A1: subcutaneously immunized rabbit with NZ9000Δ*acm*A[pNG3041] cells (recombinant, MSA2::cA surface anchored).
   B1: subcutaneously immunized rabbit with NZ9000Δ*acmA* (negative control).
   C2: orally immunized rabbit with NZ9000Δ*acmA*[pNG3043] cells (recombinant, MSA2::cP surface anchored).
   E1: orally immunized rabbit with TCA-pretreated NZ9000Δ*acmA* to which MSA2::cA had been externally bound (non-recombinant, MSA2::cA surface anchored).
   The staining bands in the lanes mean that those *L. lactis* proteins react with the indicated rabbit antiserum. It is clearly visible that the non-recombinant TCA-pretreated strain with bound MSA2::cA (E1) evokes a minimal response to *L. lactis* proteins, meaning that the response to the carrier is reduced while the response to the malaria antigen is not negatively influenced (see Table 2).

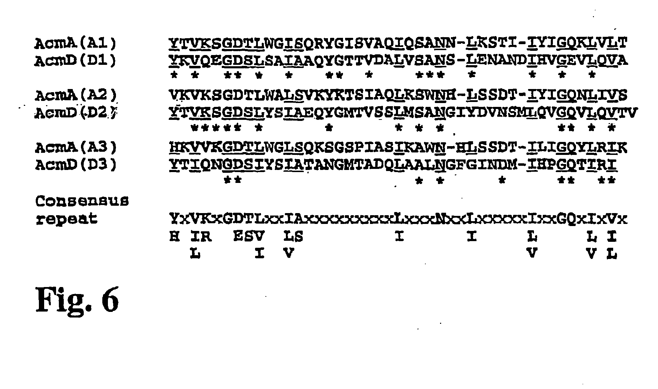

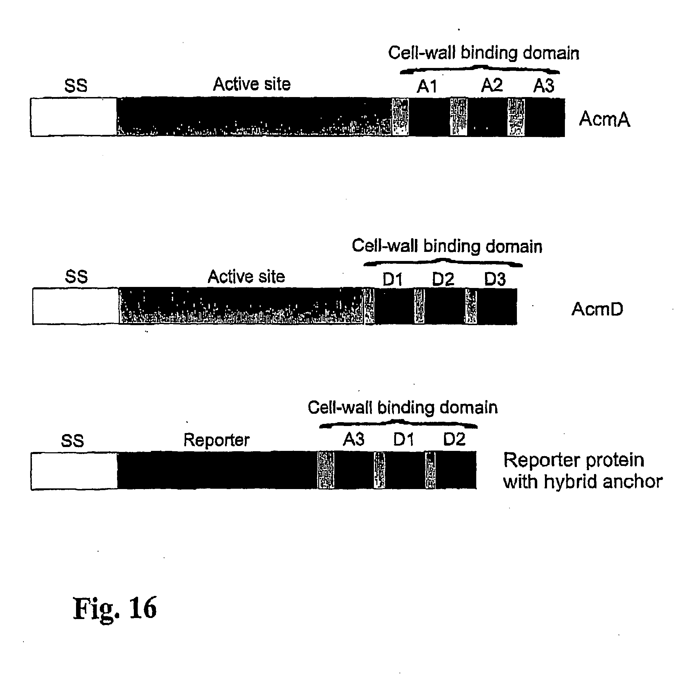

Fig. 16. Schematic representation of the domains in AcmA and AcmD. SS: signal sequence. Both enzymes have a cell wall binding domain that consists of 3 repeats indicated by A1, 2, 3 and D1, 2, 3 (the alignments of these repeats has been given in fig. 6). In addition, an example is given of one of the hybrid protein anchors described in Table 5.

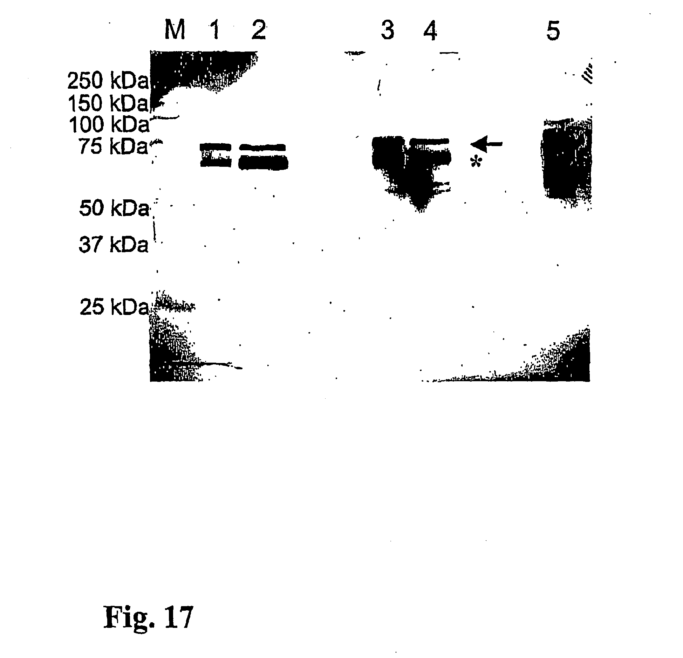

Fig. 17. Western blot that shows effect of pH supernatant on binding of MSA2::cD to TCA-pretreated *L. lactis* cells. As before, the Western blot shows the amount of MSA2::cD that was bound by the cells. In addition, the amount of MSA2::cD that was not bound and remained in the medium after binding is shown. The arrow indicates the expected position for pro-MSA2::cD and the asterisk the position of mature MSA2::cD.
   1. pH during binding 6.2, cells.
   2. pH during binding 6.2, supernatant after binding.
   3. pH during binding 3.2, cells.
   4. pH during binding 3.2, supernatant after binding.
   5. Positive control: *L. lactis,* TCA-pretreated with bound MSA2::cA at pH6.2.
   It is clearly visible that MSA2::cD binds better at pH 3.2 than at pH 6.2 (compare lanes 1 and 3).

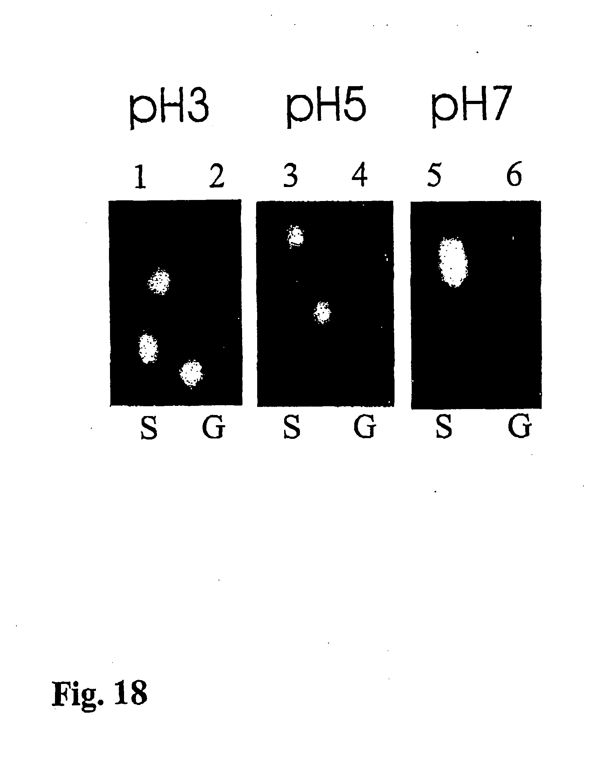

Fig. 18. Western blot of medium supernatant (S) after binding to ghost cells at the indicated pH's and ghost (G) with the bound protein anchor. Lanes 1 and 2, binding at pH3; lanes 3 and 4, binding at pH5; lanes 5 and 6, binding at pH7. The figure shows that there is still considerable binding at pH5. At this pH the native cD anchor (D1D2D3) shows little binding. The addition of the A3 repeat, which has a high pI value, results in an increase of binding at pH5.

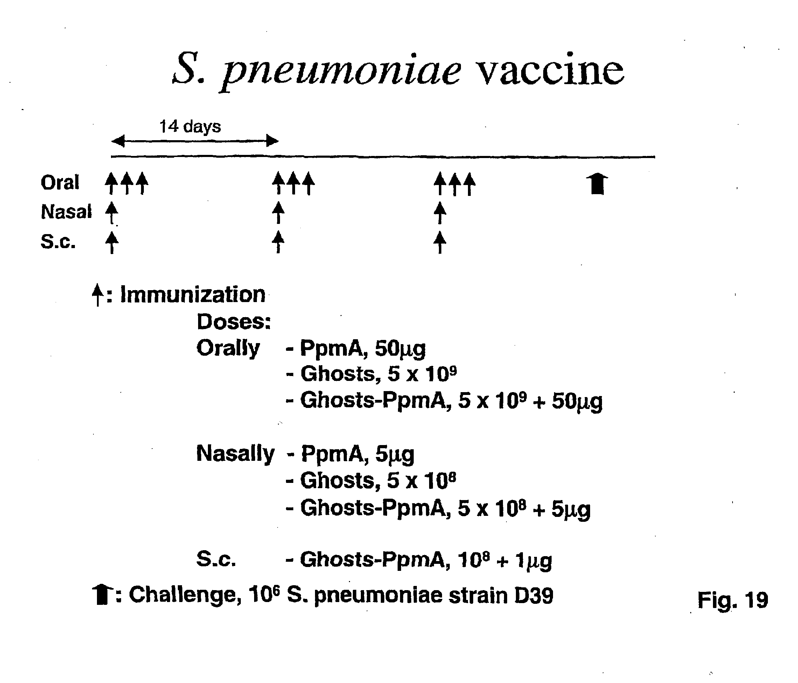

Fig. 19. Immunization schedule. Mice immunizations started at day 1 and were repeated after 14 and 28 days. A lethal nasal challenge with *S. pneumoniae* was given 14 days post the last oral immunization. S.c.: subcutaneous immunization.

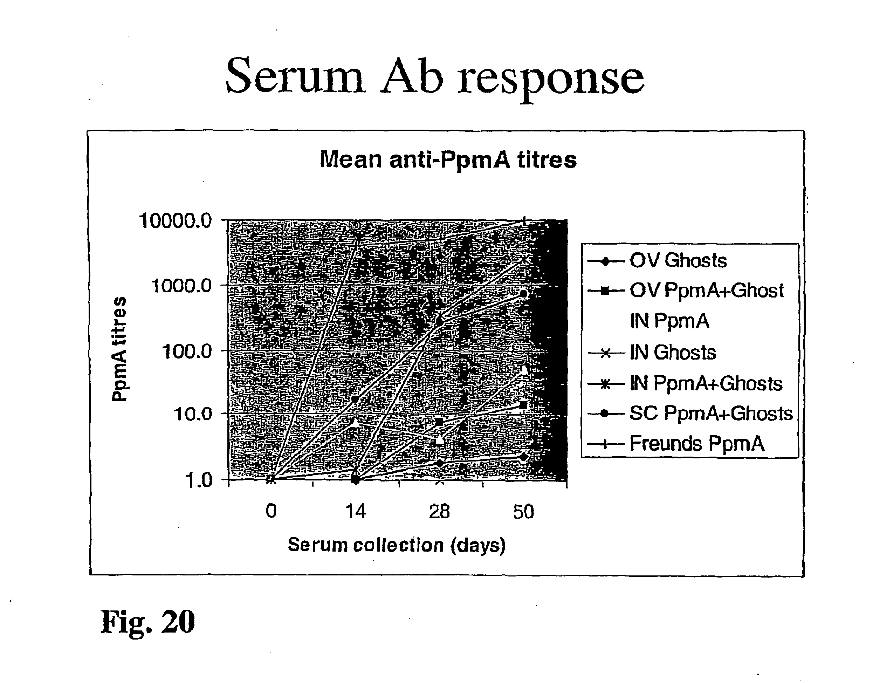

Fig. 20. Serum antibody response. Mean anti-PpmA serum antibody titers. OV: orally immunized; IN: intranasally immunized; SC: subcutaneously immunized. Freunds PpmA: soluble PpmA subcutaneously administrated together with Freunds complete adjuvants. High titers were obtained with the intranasally and subcutaneously administrated Ghosts-PpmA::cA.

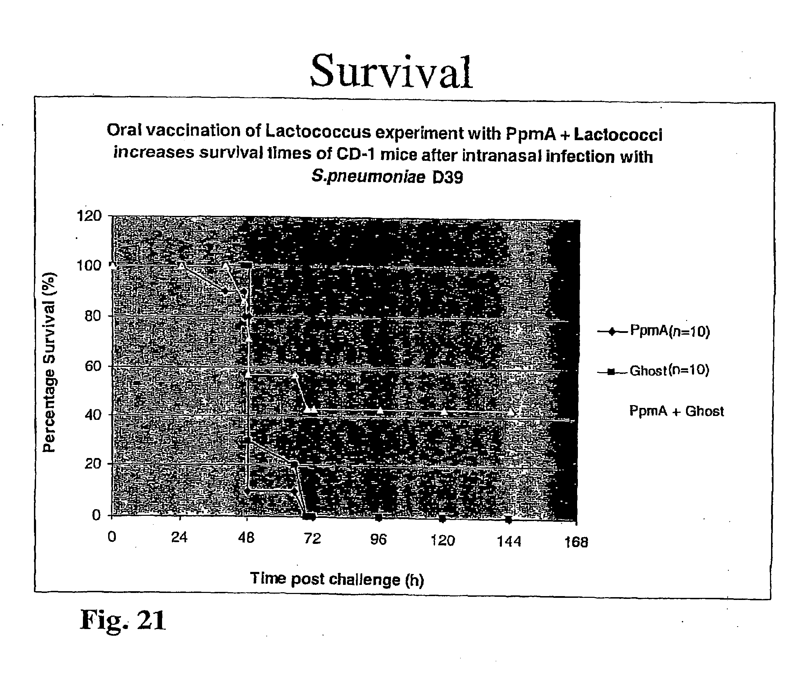

Fig. 21. Survival times. The orally vaccinated mice were challenged with a lethal dosis S. pneumonia. Mice vacinnated with soluble PpmA or Ghost alone died within 72 hours. Forty percent of the mice immunized with Ghosts-PpmA::cA survived the challenge and, therefore, were protected by the vaccination.

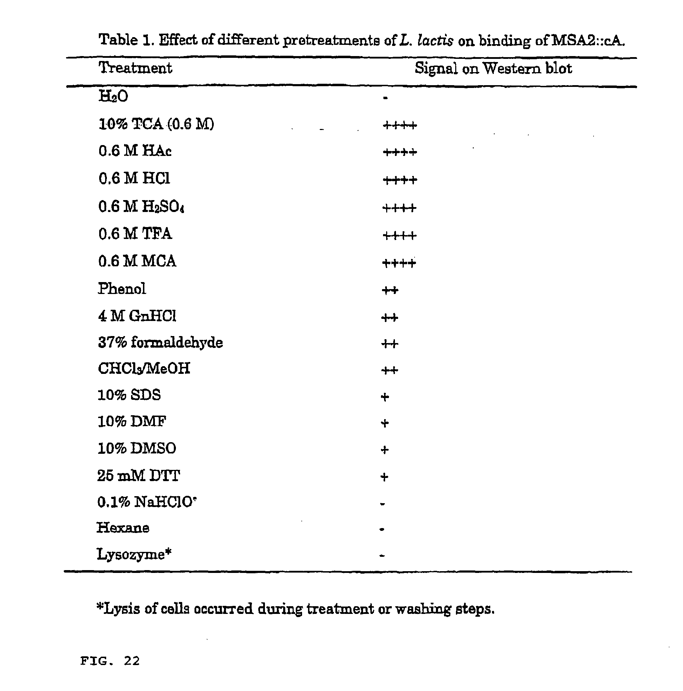

Fig. 22 is Table 1.

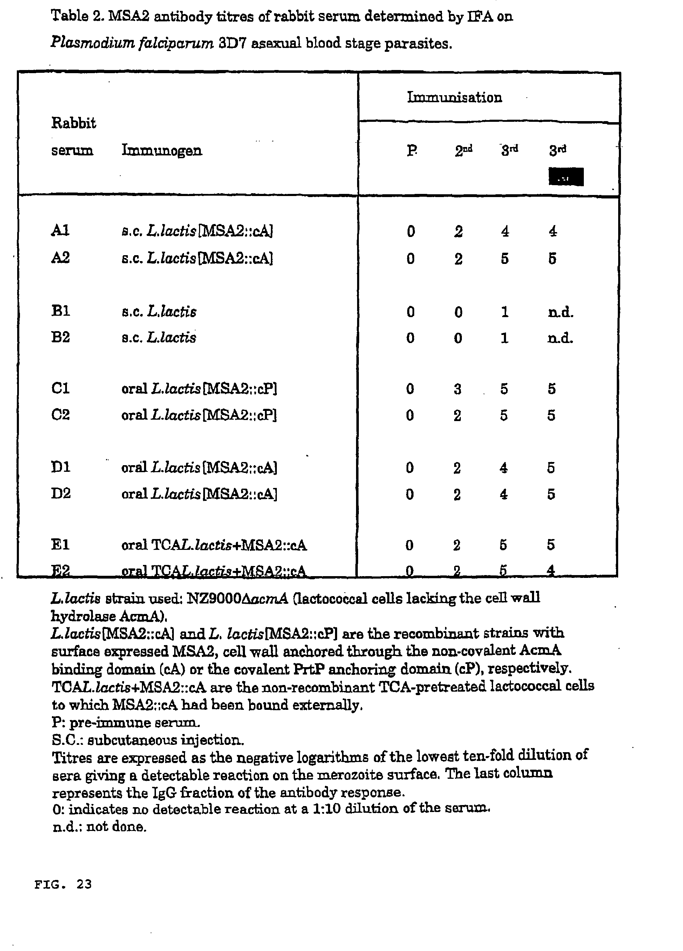

Fig. 23 is Table 2.

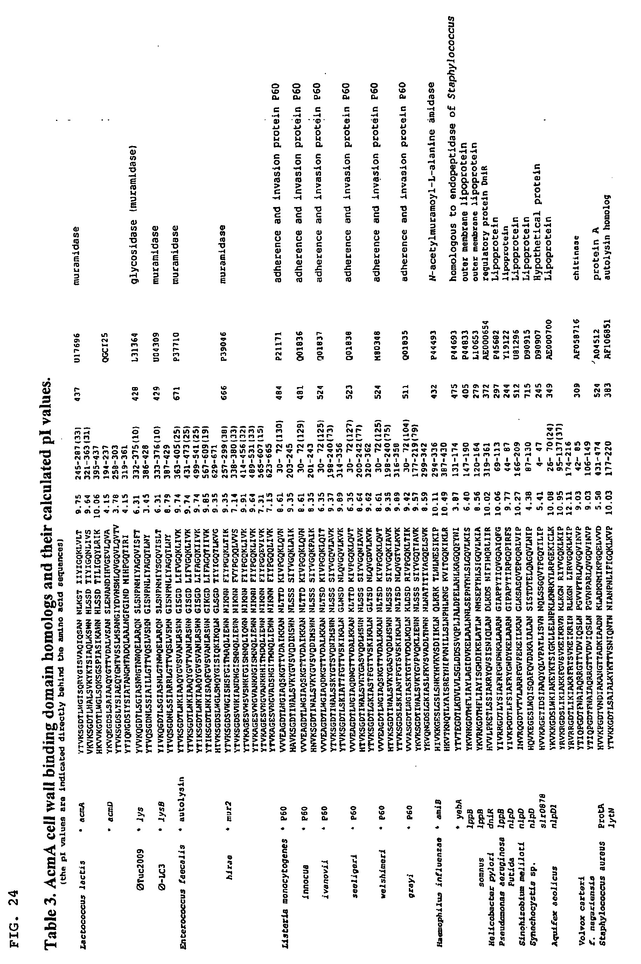

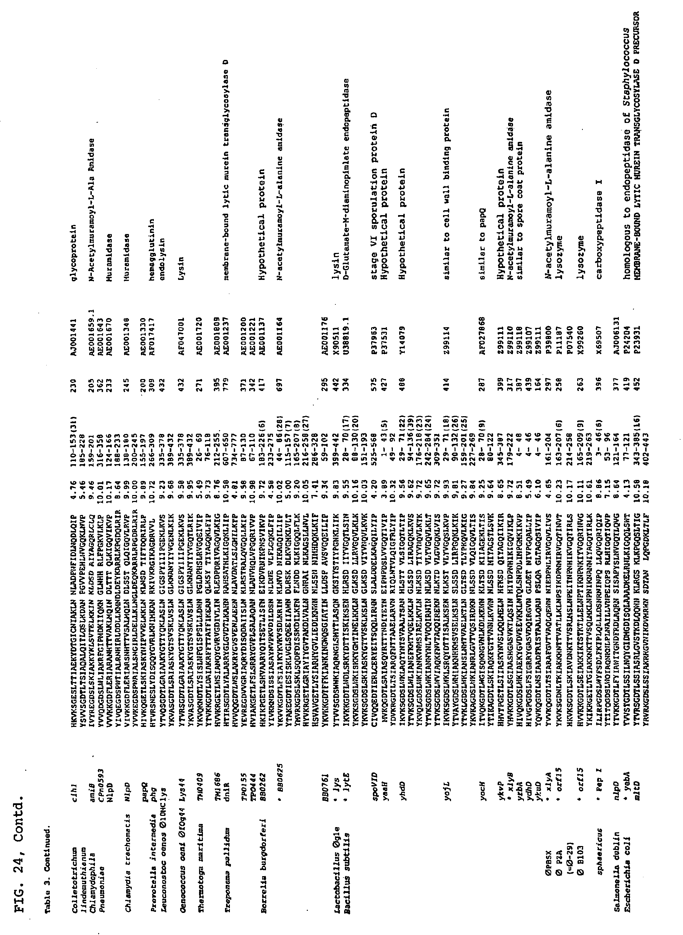

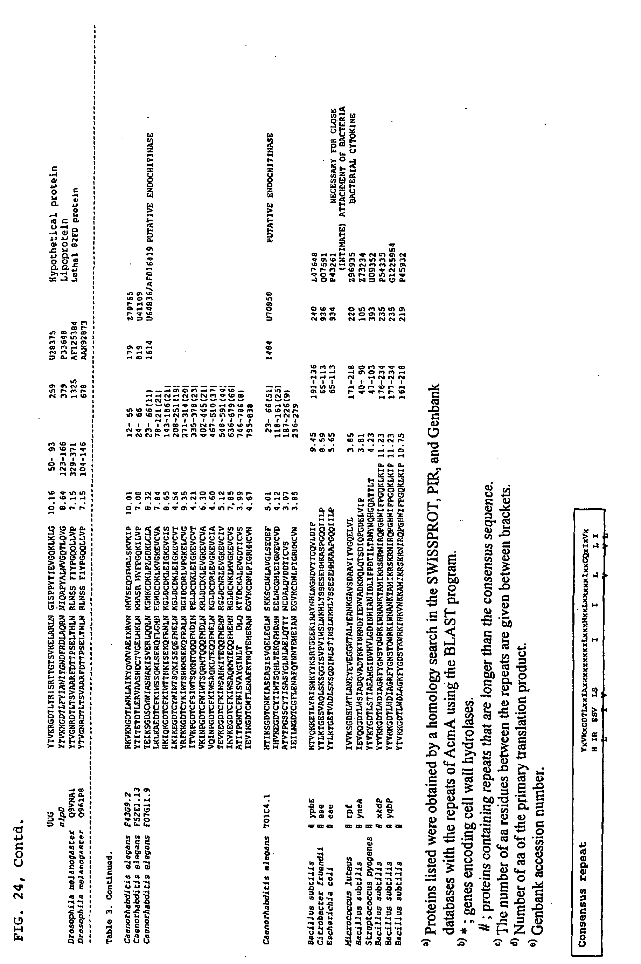

Fig. 24 is Table 3.

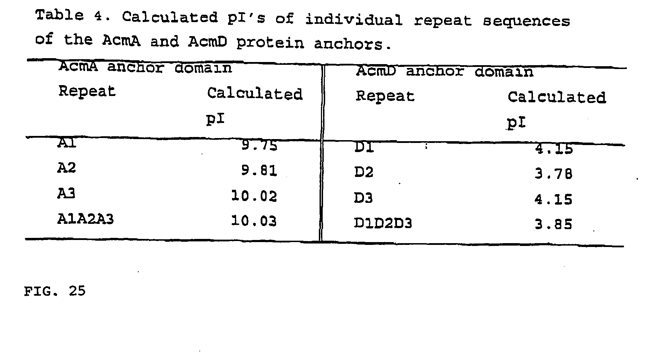

Fig. 25 is Table 4.

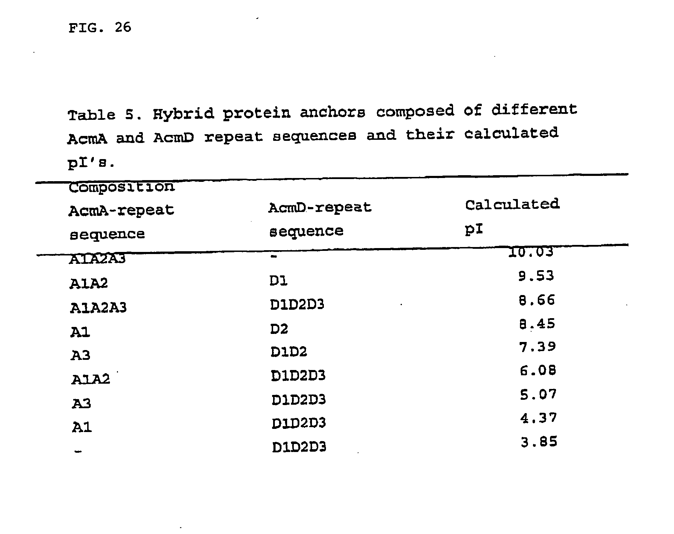

Fig 26. is Table 5.

### Detailed description

### EXAMPLE 1

### Acid pretreatment of Gram-positive bacteria enhances binding of AcmA protein anchor fusions

### Materials and Methods

*Bacterial strains and growth conditions. Lactococcus lactis* strain MG1363 (Gasson 1983) or derivatives thereof like MG1363Δ*acmA* (Buist et al. 1995) or NZ9000Δ*acmA* were used as recipients for binding of reporter fusion protein, whereas NZ9000 (Kuipers et al. 1997) carrying one of the reporter plasmids was used as production strain. *L. lactis* strains were grown in M17 broth (Oxoid) supplemented with 0.5% glucose in standing cultures at 30°C. Chloramphenicol was added to the M17 medium to an end-concentration of 5 µg/ml when appropriate. Induction for expression of mid-log phase cultures was done for 2 hr with the culture supernatant of the nisin producing *L. lactis* strain NZ9700 as described by Kuipers et al. (1997). *Lactobacillus casei* ATCC393 was grown in MRS broth (Oxoid) in standing cultures at 30°C. *Mycobacterium smegmatis* ATCC700084 was grown in Middlebrook medium (Oxoid) at 37°C in aerated cultures. *Bacillus subtilis* 168 was grown in TY broth (per liter: 10 g tryptone, 5 g yeast extract, 5 g NaCl pH7.4) at 37°C in aerated cultures.

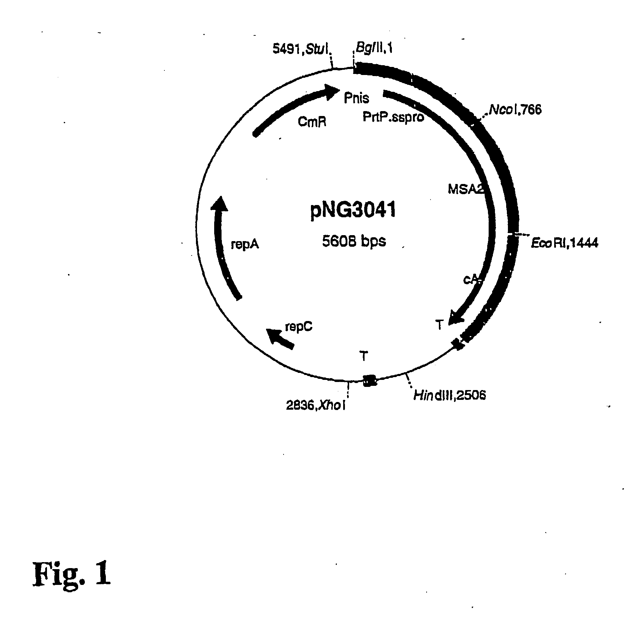

*Construction of reporter plasmids*. The merozoite surface antigen 2 (MSA2) of *Plasmodium falciparum* strain 3D7 (Ramasamy et al. 1999) fused to the three repeats of AcmA (MSA2::cA) was used as the reporter anchor protein. This reporter protein is encoded by plasmid pNG3041 that is based on the nisin inducible expression vector pNZ804S (Kuipers et al. 1997) and contains a modified multiple cloning site in which the hybrid reporter gene was cloned. An in frame fusion of this reporter was made with at the 5'-end the lactococcal PrtP signal - and prosequence, and at the 3'-end the AcmA protein anchor sequence. The sequence of the MSA2 gene that was included in the construct corresponds to nt 61 to 708 in Genbank accession number A06129. Primers that were used for the amplification of the MSA2 gene were MSA2.1 (5'-ACCATGGCAAAAAATGAAAGTAAATATAGC) and MSA2.4 (5'-CGGTCTCTAGCTTATAAGCTTAGAATTCGGGATGTTGCTGCTCC ACAG) that contain tags with restriction endonuclease recognition sites (underlined) used for cloning. For the cloning of the PrtP signal and prosequence (nt 1206 to 1766 in Kok et al. 1988) use was made of the primers PrtP.sspro.fw (5'-CCGTCTCCCATGCAAAGGAAAAAAGAAAGGGC) and PrtP.sspro.rev (AAAAAAAGCTTGAATTCCCATGGCAGTCGGATAATAAACTTTCGCC) Underlined are the restriction sites used for cloning. The AcmA protein anchor gene fragment (nt 833 to 1875) was obtained by subcloning a PvuII-*Hin*dIII fragment from plasmid pAL01 (Buist et al. 1995). Restriction endonuclease enzymes and Expand High Fidelity PCR polymerase were used according to the instructions of the supplier (Roche). The final expression vector was designated pNG3041 (Fig. 1).
A construct in which a stopcodon was introduced after the MSA2 sequence in pNG3041 was designated pNG304. The protein secreted using this construct is therefore the same as from the pNG3041 plasmid except that it does not contain the AcmA protein anchor. This is used as a negative control in the binding assays. In addition, a vector was made in which the AcmA protein anchor was exchanged for a protein anchor. For this purpose the putative cell-wall binding domain of *L. lactis* AcmD (Bolotin et al. 2001) was cloned (nt 1796 to 2371 in Genbank accesssion number AE006288) using primers pACMB2 (5'-CGCAAGCTTCTGCAGAGCTCTTAGATTCTAATTGTTTGTCCTGG)and pACMB3 (5'- CGGAATTCAAGGAGGAGAAATATCAGGAGG). The resulting plasmid, pNG3042, contains an in frame fusion between MSA2 and the protein anchor of AcmD (MSA2::cD) and differs only in the gene fragment encoding the protein anchor from plasmid pNG3041.

*Cell pretreatment and binding conditions*. Chemical pretreatment of *L. lactis* NZ9000Ä*acmA* was routinely done with 10% TCA (0.6 M) as follows: cells of 0.5 ml stationary phase cultures were sedimented by centrifugation and washed once with 2 volumes demineralized water. Cells were resuspended in 1 volume of a 10% TCA solution and incubated by placing the reaction tube for 15 min in boiling water. Subsequently, cells were washed once with 2 volumes PBS (58 mM Na₂HPO₄•2H₂O, 17 mM NaH₂PO₄•H₂O, 68 mM NaCl; pH 7.2) and three times with 2 volumes demineralized water. Cells prepared in this way were used directly for binding experiments or stored (see next paragraph) until further use. The following chemicals and conditions were also used to examine the effect of different chemicals on the binding capacity of *L. lactis* cells for AcmA-type protein anchor fusions: acetic acid (HAc), hydrochloric acid (HCl), sulphuric acid (H₂SO₄), TCA, trifluoric acid (TFA), monochloric acid (MCA), all at a final concentration of 0.6 M and 15 min incubation in boiling water. SDS, dimethyl formamide (DMF) and dimethyl sulfoxide (DMSO) were all used at a concentration of 10% combined with the 15 min incubation in boiling water for the SDS pretreatment and at room temperature for the DMF and DMSO treatments. Cells were also pretreated with phenol (Tris buffer saturated) and then incubated for 15 min at 55° C. Other chemicals used at the latter incubation temperature were: 4 M guanidine hydrochloride (GnHCl), 37% formaldehyde, chloroform:methanol (CHCL₃:CH₃OH [2:1]) and 0.1% sodium hypochlorite (NaOCl). In addition, incubation with 25 mM dithiothrietol (DTT) for 30 min at 37°C and a pretreatment with hexane (100%) were analyzed. The effect of enzymatic pretreatment of cells with lysozyme was also tested. For this purpose cells were resuspended in buffer (20% sucrose, 10 mM Tris pH8.1, 10 mM EDTA, 50 mM NaCl) with lysozyme (2 mg/ml) followed by incubation at 55°C for 15 min. The washing steps after the chemical and enzymatic pretreatments were the same as for the TCA treated cells.
TCA pretreatment of *Bacillus subtilis, Lactobacillus casei* and *Mycobacterium smegmatis* was done as described for *L. lactis.*
Cell-free culture supernatants containing MSA2::cA, MSA2::cD or MSA2 without anchor were incubated in four-fold excess for 10 min at room temperature with (pretreated) cells (e.g. cells from 0.5 ml culture were incubated with 2.0 ml culture supernatant). After binding, cells were sedimented by centrifugation, washed twice in 2 volumes demineralized water, resuspended in SDS-denaturation buffer, heated for 5 min at 98°C and subjected to SDS-PAGE followed by Western blot analysis.

*Storage conditions*. Cell-free supernatants containing MSA2::cA, MSA2::cD or MSA2 were stored prior to binding at -20°C with or without 10% glycerol. TCA pretreated *L. lactis* cells were stored prior to binding at -80°C in 10% glycerol. TCA pretreated *L. lactis* cells with bound MSA2::cA were stored at +4°C or -80°C with or without 10% glycerol. Cells that were stored in 10% glycerol were washed once with 1 volume of demineralized water prior to binding.
Cell pellets (in demineralized water) of TCA pretreated *L. lactis* cells with or without bound MSA2::cA were frozen by contacting the vials to liquid nitrogen and, subsequently, water was removed by lyophilization. Alternatively, non-frozen cell pellets were dried under vacuum at 30°C for 2 h prior to binding.

*Western blotting*. For detection of MSA2 proteins, cell pellets corresponding to 500 µl culture were resuspended in 50 µl SDS-denaturation buffer. Cell-free culture supernatants (1 ml) were concentrated by phenol-ether precipitation (Sauvé et al. 1995), vacuum dried and resuspended in 50 µl SDS-denaturation buffer. Proteins were separated by using standard SDS-PAGE techniques. After separation, proteins were electroblotted onto PVDF membranes (Roche). In immunoblots, MSA2 proteins were detected with 1:10,000 diluted rabbit MSA2-specific antiserum (Ramasamy et al. 1999) and 1:5,000 diluted anti-rabbit IgG-conjugated alkaline phosphatase (Roche) using standard procedures.

*Fluorescence microscopy*. Cells suspensions of 100 µl incubated with MSA2::cA, MSA2::cD or MSA2 fusion proteins, were washed twice with demineralized water and resuspended in an equal volume PBS containing 1% BSA and 1:200 diluted MSA2-specific rabbit antiserum. After incubation for 20 min at room temperature, the cells were washed three times with 2 volumes PBS. Subsequently, the cells were incubated 20 min in 1 volume PBS with 1% BSA and 1:100 diluted Oregon green labeled goat anti-rabbit immunoglobulin G (Molecular Probes). After washing once with 2 volumes PBS and twice with 2 volumes demineralized water, the cells were resuspended in 100 µl demineralized water. A 10 µl aliquot of this cell suspension was spread onto a Polysin microslide (Menzel-Gläser), air dried, and examined under a fluorescence microscope (Zeiss).

*Electron microscopy*. TCA-pretreated *L. lactis* cells incubated with MSA2::cA, MSA2::cD or MSA2 were collected and washed as described above. The immunogold labeling was performed on whole mount preparations of glutaraldehyde fixed cells on Formvar-carbon coated nickel grids using Auroprobe 15 nm goat anti-rabbit IgG gold marker (Amersham). The primary antibodies against MSA2 were diluted 1: 1000 in PBS-glycine buffer. Subsequently, the labeled samples were stained with 0.1% uranyl acetate (W/V in water) and examined in a Philips CM10 transmission electron microscope at 100 kV..

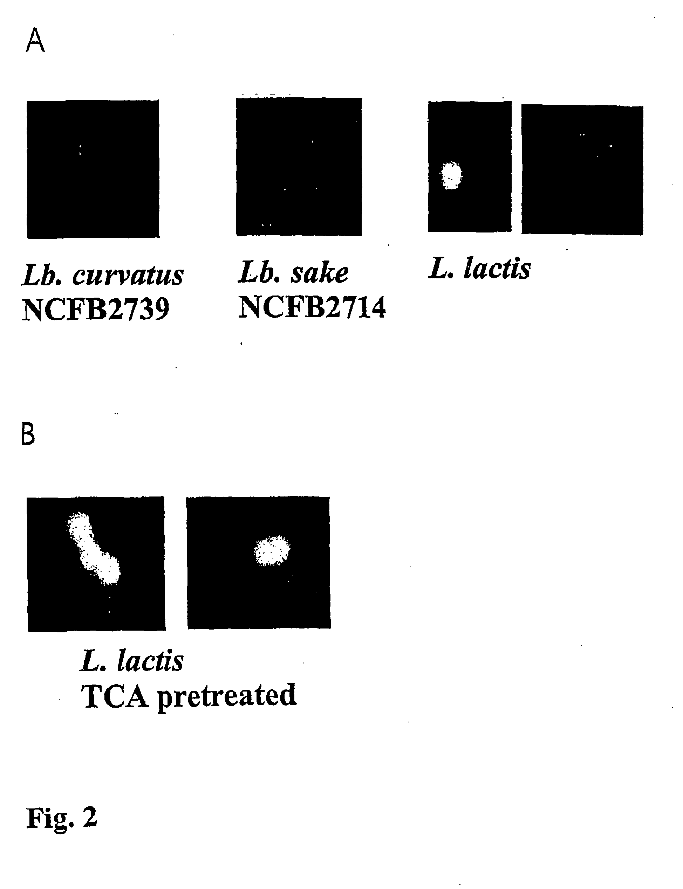

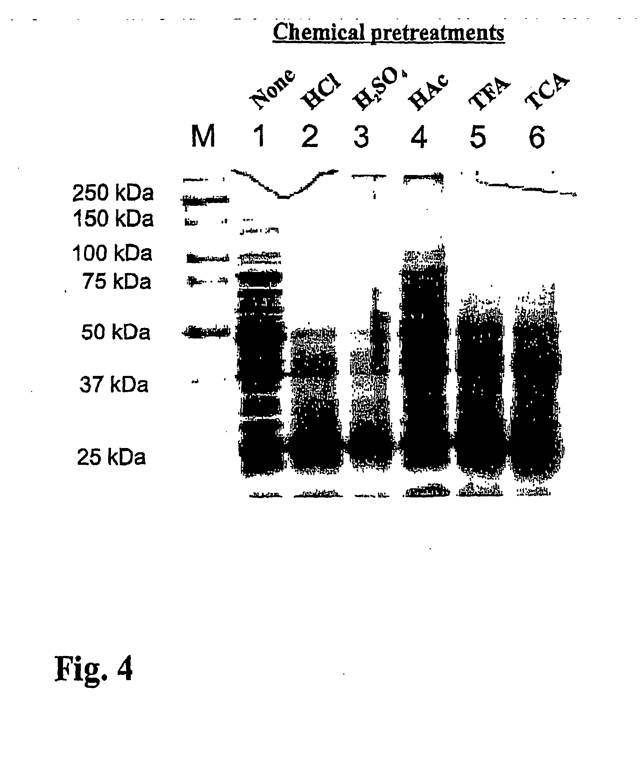

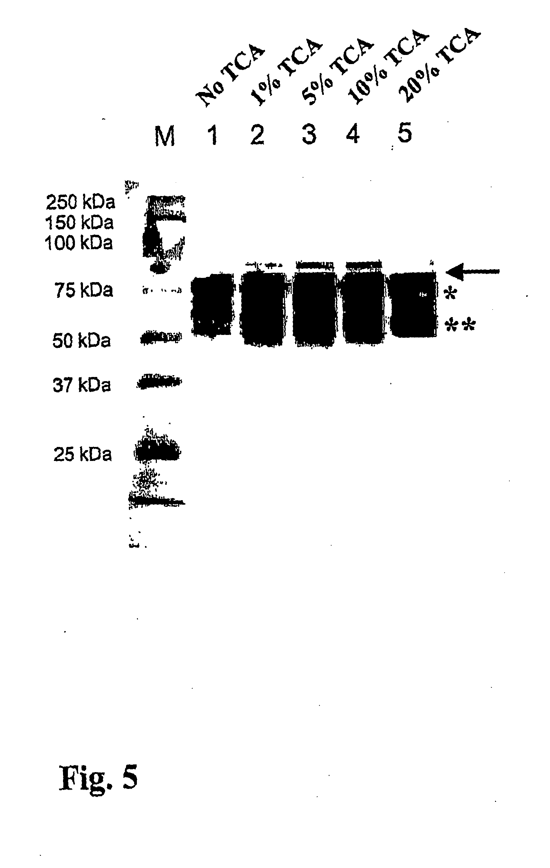

*Pretreatment of L. lactis cells with different chemicals*. The cA protein anchor of *L. lactis* AcmA can be used to bind fusion proteins to a wide variety of Gram-positive bacteria. However, the amount of fusion protein that binds varies greatly among this group of bacteria. We observed binding of MSA2::cA that covers the entire cell surface of some lactobacilli, whereas other bacteria such as *L. lactis* show only limited localized binding (Fig. 2A). An explanation for this phenomenon could be, although other explanations are possible, that the cell walls of some bacterial species contain components that interfere with cA anchor binding. Chemicals like SDS, TCA, chloroform/methanol and others are used to remove components from isolated bacterial cell walls (Morata de Ambrosini et al. 1998). Therefore, we investigated the effect of removal of cell wall components from *L. lactis* whole cells on the binding of the reporter fusion protein MSA2::cA. *L. lactis* cells were pretreated as described in the Materials and Methods with various chemicals or with lysozyme. Fig. 3 shows typical Western blots of pretreated whole cells to which MSA2::cA was bound. Mature MSA2::cA migrates at a position of a 75 kDa protein (indicated by an asterisk). The arrow represents MSA2::cA that still contains the PrtP prosequence. The double asterisks represents MSA2::cA from which one or two of the repeats have been removed. A cell membrane anchored protease HtrA has been shown to be involved in processing proproteins and in removing repeats from AcmA (Poquet et al. 2000). From Fig. 3 can be concluded that pretreatment with TCA (lanes 8 and 16 contain the same samples, the difference in signal intensity is due to differences in stain developing time), HCl, H₂SO₄ and HAc improves the subsequent binding of MSA2::cA (compare with the negative control in lane 15) substantially. Other acids that were tested, TFA and MCA, had similar effects (not shown). Phenol, GnHCl, formamide and chloroform/methanol pretreatments showed a moderate improvement of binding (lanes 4, 5, 6, 7, respectively). Minor binding improvements were observed after pretreatment with SDS, DMF, DMSO and DTT. All the results are summarized in Table 1. We conclude that pretreatment of *L. lactis* cells with the acids TCA, TFA, MCA, HCl, H₂SO₄ and HAc are the most effective agents to improve binding of cA anchor fusion proteins to lactococcal cells. Acids like TCA are known to remove (lipo)teichoic acids from cell walls. We also analyzed whether proteins are removed from the cell walls by these acid treatments. Fig. 4 shows a Coomassie stained gel of lysed pretreated cells. Most of these acid treatments, except for HAc, remove substantial amount of proteins from the lactococcal cells. Since HAc, removes only trace amount of proteins (compare lane 1 and 4) and SDS pretreatment (which is known to remove proteins from the cell walls) showed only a minor improvement of MSA2::cA binding (Fig. 3, lane 1), we conclude that removal of proteins from the cell wall is not critical for binding improvement of cA anchor fusions. Most likely is that lipoteichoic acids or carbohydrates occupy sites in the cell walls of *L. lactis* that interfere with efficient binding. Alternatively, acid pretreatment may result in altering the compactness of peptidoglycan strands that makes cA binding sites more available.
TCA pretreatment was used in all other experiments. Next, we determined the optimal TCA concentration in the boiling procedure. Percentages of 1, 5, 10 and 20% were tested. Although 1% TCA pretreatment showed already a significant improvement in binding of MSA2::cA and 5% TCA showed an further increase, no further improvement was observed at concentrations higher than 10% (Fig. 5). We selected therefore the boiling procedure with 10% TCA as the standard procedure for all further experiments.
Subsequently, we analyzed the binding characteristics of the lactococcal cA hoinolog cD in a MSA2 fusion using the standard TCA pretreatment procedure. Two of the three AcmD repeats are highly homologous to those of AcmA, an alignment is given in Fig. 6. A negative control, secreted MSA2 without anchoring domain was included in these experiments. In Western blots, the effect of TCA pretreatment on the binding of MSA2::cA is evident (Fig. 7, compare lanes 1 and 4). This was also studied using fluorescence microscopy (Fig. 2, compare *L. lactis* in A and B; Fig. 8) and electron microscopy (Fig. 9, compare A and B). Independent of the technique used the effect of TCA pretreatment on the binding of MSA2::cA can be clearly detected. Binding of MSA2::cD to non-TCA pretreated *L. lactis* cells was low as detected in Western blots (Fig. 7, lane 2), but was undetectable in fluorescence microscopy and electron microscopy (Fig. 9A). TCA pretreatment had only minor effects on the intensity of the MSA2::cD signal in Western blots (Fig. 7, lane 5). At the same time no MSA2::cD specific signal associated with the pretreated cells could be observed in fluorescence microscopy (Fig. 8) and only low levels of labeling in electron microscopy (Fig. 9C). Surprisingly, some cell-associated signal was observed for MSA2 without anchoring domain for both non-TCA pretreated and TCA pretreated *L lactis* cells (Fig. 7, lanes 3 and 6, respectively). However, as for MSA2::cD this could not be seen in fluorescence microscopy (not shown) and only minor labeling signals were found in electron microscopy (Fig. 9D). Taken together, we conclude that: (i) the reporter protein MSA2 does have some low degree of affinity for bacterial cell walls that can be detected in Western blots; (ii) the cA anchor domain stimulates specifically the binding of the reporter fusion to non-pretreated cells, iii) chemical pretreatment, especially with acids enhances this binding and; (iv) the cD anchor domain does not promote binding of fusion proteins under the conditions applied.
The fluorescence- and electron microscopic images of TCA pretreated lactococcal cells (Fig. 2, 8 and 9) clearly showed that the pretreatment leaves the cell integrity intact. However, cells are no longer viable (plating efficiency 0) and can therefore be considered as inert spherical peptidoglycan microparticles with a diameter of approx. 1 µm ('ghost cells').

*Binding to other Gram.-positives*. The binding of MSA2::cA, MSA2::cD and MSA2 without anchor domain to the Gram-positive bacteria *B. subtilis, Lb. casei* and *M. smegmatis* was also analysed. Fig. 10 shows a Western blot that summarizes the binding to non-pretreated and TCA-pretreated *B. subtilis* cells. As for *L. lactis* a clear increase in binding is observed for MSA2::cA. A MSA::cA specific signal could also be visualized in fluorescence microscopy of non-pretreated *B. subtilis* cells, but with a highly improved signal for the TCA-pretreated cells (not shown). Binding of MSA2::cD and MSA2 to non-pretreated or TCA-pretreated cells could not be demonstrated in fluorescence microscopy (not shown).
Similar results were obtained for *Lb. casei* and *M. smegmatis.* The improved binding of MSA2::cA to TCA-pretreated *Lb. casei* cells is shown in Fig. 11. For MSA2::cD and MSA2 no fluorescence signals were detected (not shown). The TCA-pretreatment of *M. smegmatis* had also a positive effect on the binding of MSA2::cA, whereas no binding was observed for MSA2::cD or MSA2 (Fig. 12).
Taken together, we conclude that acid pretreatment, such as with TCA, improves the binding of cA protein anchor fusions to the cell surface of Gram-positive bacteria.

*Binding strength and storage conditions*. To analyze the strength of the MSA2::cA binding to TCA-pretreated *L. lactis* cells, a treatment with LiCl was carried out after the binding LiCl is commonly used to remove proteins from bacterial cell walls. From the Western blot in Fig. 13 it was concluded that 8 M LiCl only partially removes MSA2::cA from the *L. lactis* cells (compare lanes 4 and 5). Therefore, although MSA2::cA binds non-covalently to cell walls, the binding interactions must be very strong.
Cell-free culture supernatants with MSA2::cA was stored with or without 10% glycerol at -20°C. MSA2::cA stored in this way for several weeks still had the same capacity to bind to TCA-pretreated *L. lactis* cells (not shown). TCA-pretreated *L. lactis* cells with bound MSA2::cA were stored for 3 weeks at +4°C in demineralized water or at -80°C in demineralized water with or without 10% glycerol. The samples were all analyzed in Western blots. Storing pretreated cells with bound MSA2::cA for 3 weeks in water at +4°C resulted only in a loss of signal of about 50% (Fig. 13, lane compare lanes 4 and 6). Whether this loss of signal was due to degradation or to release of the protein into the water was not determined. Storage at -80°C with or without 10% glycerol had no effect on the binding (Fig. 13, compare lanes 4, 7 and 8).

In addition, we studied the effects of drying and lyophilization on the binding of MSA2::cA to TCA-pretreated *L. lactis* cells. Drying of pretreated cells had no negative effect on binding of MSA2::cA afterwards. Dried pretreated cells with bound MSA2::cA could be resuspended in water without loss of bound fusion protein. This was also the case for lyophilized cells with bound MSA2::cA. Lyophilization of TCA-pretreated cells prior to binding, resulted in loss of the binding capacity for MSA2::cA (results not shown).
From these data we conclude that: (i) in spite of the non-covalent character of cA anchor binding to cell walls, this binding is very strong; (ii) cell-free culture supernatants can be stored safely at -20°C and, (iii) drying of TCA-pretreated cells provides an efficient and simple method for storage of such cells either with or without bound cA-anchor fusions.

### EXAMPLE 2

### Oral immunizations of rabbits with non-recombinant Lactococcus lactis preloaded with the Plasmodium falciparum malaria antigen MSA2 fused to the lactococcal AcmA protein anchor

In Example 1 a technology is described to efficiently bind protein hybrids, when added externally, to the cell surface of non-recombinant gram-positive bacteria by means of an AcmA-type protein anchor. Therefore, this technology provides the possibility to provide bacteria or bacterial cell walls with new traits without introducing recombinant DNA into them. Here we investigated the immunogenicity in rabbits of the *Plasmodium falciparum* merozoite surface protein, MSA2 of strain 3D7 (Ramasamy et al. 1999), presented on the cell surface of non-recombinant non-living *L. lactis* cells as an AcmA anchor fusion protein.

### Materials and Methods

*Bacterial strains and growth* conditions. The *L. lactis* strain producing MSA2::cA, its growth conditions, the induction for expression, the TCA pretreatment of the *L. lactis* recipient cells and the binding of MSA2::cA to these cells was as described in example 1 with the following modification: a ratio of 1 (TCA-pretreated cells) to 5 (cell-free culture supernatant with MSA2::cA) was used for binding. As a positive control in the immunization experiments an *L. lactis* NZ9000 strain carrying plasmid pNG3043 was used (was positive in a previous unpublished experiment). This plasmid encodes an MSA2 hybrid protein that contains at its C-terminus the lactococcal PrtP cell wall anchoring domain (MSA2::cP) instead of the AcmA protein anchor. The PrtP cell wall anchoring domain contains the LPXTG motive that enables a membrane-linked sortase to covalently couple the protein to the cell wall (Navarre and Schneewind 1994). The cP domain used in construct pNG3043 corresponds to nt 6539 to 6914 in Kok et al. (1988). Primers used for the amplification of this fragment were PrtP.cwa.fw3 (5'-ATATAAAGCTTGCAAAGTCTGAAAACGAAGG) and PrtP.cwa.rev (5'-CCGTCTCAAGCTCACTATTCTTCACGTTGTTTCCG). Underlined are the restriction endonuclease recognition sites used for cloning. Plasmid pNG3043 differs only in the cell wall binding domain from plasmid pNG3041. Growth conditions and induction of expression of strain NZ9000Δ*acmA*[pNG3043] were the same as for strain NZ9000Δ*acmA*[pNG3041].

*Rabbit immunisations*. Ten barrier-reared, New Zealand white rabbits obtained from Harlan laboratories, The Netherlands were used in groups of 2 for experimental immunizations. The care and use of animals were according to WHO guidelines (WHO/LAB/88.1). The rabbits were ear bled prior to immunisation to obtain preimmune sera. Details of the rabbits and immunogens are as follows:
Rabbits A1 and A2, subcutaneously immunized with NZ9000Δ *acmA*[pNG3041] cells (recombinant, MSA2::cA partly surface anchored).
Rabbits B1 and B2, subcutaneously immunized with NZ9000Δ*acmA* (negative control).
Rabbits C1 and C2, orally immunized with NZ9000Δ*acmA*[pNG3043] cells (recombinant, MSA2::cP surface anchored).
Rabbits D1 and D2, orally immunized with NZ9000Δ*acmA*[pNG3041] cells (recombinant, MSA2::cA surface anchored).
Rabbits E1 and E2, orally immunized with TCA treated NZ9000Δ*acmA* to which MSA2::cA had been bound from NZ9000Δ*acmA*[pNG3041] culture supernatant (non-recombinant, MSA2::cA surface anchored).
Stocks of NZ9000Δ*acmA*[pNG3043] with MSA2::cP expressed at its surface stored in aliquots of 10¹¹ cells in growth medium containing 10% glycerol at - 80°C. The cells remain viable under these conditions and retain MSA2 on the surface as demonstrated by immunofluorescence (not shown). The first immunization was carried out with freshly grown bacteria. For subsequent immunizations, stocks of bacteria were freshly thawed, washed and resuspended in buffer at the appropriate concentration for immunizations.
On the other hand, the non-pretreated NZ9000Δ*acmA* (negative control), the non-pretreated NZ9000Δ*acmA*[pNG3041] and the TCA-pretreated NZ9000Δ *acmA* with the externally bound MSA2::cA were daily prepared from fresh cultures.
Subcutaneous injections were performed with a total of 5 x 10⁹ cells in 100 ìl PBS without any adjuvant into two sides on either side of the spine The subcutaneous injections were repeated twice more at 3 week intervals. Prior to oral immunization, the rabbits were deprived of water and food for 2-4h.
They were then fed 5 x 10¹⁰ cells resuspended in 1ml of 0.5% sucrose. Each dose was repeated for three successive days to obtain reproducible oral immunization. Altogether three series of oral immunizations were given at 3 week intervals. Adverse effects consequent to the immunizations, including granulomas at the sites of subcutaneous injections, were not observed indicating that *L. lactis* was well tolerated by the animals.

*Serum antibody responses*. Rabbits were ear bled 2 weeks after each immunization to obtain sera for antibody assays. The sera were stored at - 20°C until use. Ten-fold serial dilutions of the antisera in 2% BSA in PBS were used in immunofluorescence assays (IFA) to determine the titre of the antibodies against MSA2 on the surface of 3D7 *P*. *falciparum* merozoites. IFA was performed on acetone-methanol fixed late stage 3D7 *P*. *falciparum* parasites as previously described (Ramasamy 1987). For detection of all antibody isotypes, Oregon Green conjugated goat anti-rabbit Ig (Molecular Probes) was used as the second antibody. For detection of IgG antibodies only, a fluorescein conjugated, affinity purified, mouse monoclonal with specificity against rabbit ã chains (Rockland), was used instead.

### Results and Discussion

*Surface expression of MSA2 in different L. lactis strains*. Coomassie staining of SDS-PAGE gels and fluorescence microscopy were used to determine in a semi-quantitative way the number of MSA2 molecules expressed and surface exposed by the recombinant lactococcal strains carrying plasmid pNG3041 or pNG3043 that produce MSA2::cA or MSA2::cP, respectively, and by the non-recombinant TCA-pretreated *L. lactis* cells to which MSA2::cA had been bound from the outside. We estimated that the recombinant strains produced approximately 1.4 x 10⁵ molecules of MSA2::cA or MSA2::cP. Surface exposure of MSA2::cA and MSA2::cP, however, differed considerably as shown by fluorescence microscopy in Fig. 14. The non-recombinant TCA-pretreated *L. lactis* cells with bound MSA2::cA showed a uniform staining of the entire cell surface. However, the semi-quantitative SDS-PAGE analysis indicated that this represents about 1 x 10⁴ molecules of MSA2::cA per cell. Therefore, we conclude that the number of surface exposed MSA2::cA and MSA2::cP on the recombinant lactococcal strains is less than 10% of total number of molecules produced by these strains. The other molecules are most likely trapped in the membrane or cell wall. Similar observations were made by Norton et al. (1996) for the expression of TTFC fused to the cP cell wall anchoring domain. In that study only membrane- or cell wall-associated but no surface exposed TTFC::cP could be demonstrated. It seems therefore that binding from the outside to TCA-pretreated cells is a more efficient method to surface-expose proteins on *L. lactis* cells.

*Anti-MSA2 antibody responses in orally immunized rabbits.* Characteristics of the anti-MSA2 antibody response to the immunizations are summarized in Table 2. The oral immunizations with the recombinant *L. lactis* that produce MSA2::cP (rabbits C1 and C2) were done once before (unpublished results) and served here as a positive control. In the previous experiment a similar antibody response was found. The present experiment showed that specific antibodies against near native MSA2 were also detectable after two immunisations for group A, D and E rabbits, and that antibody titres increased after a third immunisation, in all instances. IgG antibodies were predominant after three immunisations by either the subcutaneous or oral route. A comparatively weak anti-MSA2 surface IFA, attributable to the generation of cross-reactive antibodies (see below), was also observed after three control subcutaneous immunisations with *L. lactis* cells alone.
Taken these results together, we conclude that: (i) MSA2 produced by lactococcal cells elicits serum antibodies that recognize native *P*. *falciparum* parasite MSA2, (ii) MSA2-specific Tₕ cells are activated through mucosal immunization due to the presence systemic IgG antibodies (Table 2) that can be boosted (unpublished results), and (iii) oral immunizations with MSA2::cA bound to non-recombinant non-living TCA-pretreated *L. lactis* cells are as efficient in evoking specific serum antibody responses as the live recombinant strain producing MSA2::cA that was administered either subcutaneously or orally, or the live recombinant strain producing MSA2::cP that binds MSA2 covalently to its cell wall and was delivered orally.

*Anti-lactococcal antibody responses*. Western blots (Fig. 15) demonstrated significant antibody responses against *L. lactis* antigens after two and three immunisations of the rabbits. The responses were notably greater after subcutaneous (group A and B rabbits) than oral immunization with *L. lactis* (group C rabbits). Oral immunization with the TCA-pretreated lactococcal cells (group E rabbits) elicited antibodies that reacted at a lower intensity with fewer *L. lactis* antigens than oral immunisation with viable *L*. *lactis* cells. Most likely, this is due to the fact that proteins are removed from the lactococcal cells by the TCA pretreatment (see example 1). The lower anti-carrier response observed for the TCA-pretreated (non-recombinant) cells renders this type of delivery vehicle more suitable for repeated immunization strategies than its untreated (recombinant) counterpart.

### EXAMPLE 3

### pH Dependent cell-wall binding of AcmA protein anchor homologs and hybrids

The cell wall binding domain or anchor of the lactococcal cell wall hydrolase AcmA consists of three repeats of 45 amino acids that show a high degree of homology (Buist et al. 1995). These repeats belong to a family of domains that meet the consensus criteria as defined in patent application WO99/25836 and can be found in various surface located proteins in a wide variety of organisms. Another feature that most of these domains have in common is that their calculated pI values are high: approximately 8 or higher (Table 3). The pH that was used by us in previous binding experiments with MSA2::cA (example 1 and 2) was approx. 6, meaning that the binding domain was positively charged. The AcmA protein anchor homolog of the lactococcal cell wall hydrolase AcmD [cD] (Bolotin et al. 2001) consists also of three repeats (Fig. 16) with a calculated pI that is much lower (approximately pI 3.8) than that of the cA domain (Table 4). Consequently, the cD anchor was negatively charged at the binding conditions used in example 1. We have demonstrated that no binding of the MSA2::cD reporter protein occurred under these conditions. Therefore, we investigated here the influence of the pH during binding of a cD fusion protein (MSA2::cD). Furthermore, we constructed a hybrid protein anchor consisting of the three cD repeats and one cA repeat that has a calculated pI value that is higher than that of the cD repeats alone. The hybrid protein anchor showed better binding pH values above the pI of the cD repeats alone, indicating that the pH binding range of AcmA-type protein anchors can be manipulated by making use of the pI values of the individual repeats in hybrids.

### Materials and Methods

Bacterial strains, growth and induction conditions, TCA pretreatment of *L. lactis* cells, incubation of the MSA2 protein anchor fusion proteins to TCA-pretreated cells, washing conditions, protein gelelectrophoresis, Western blotting and immunodetection were the same as described in example 1. Under the conditions used, the cell-free culture supernatants with MSA2::cA, MSA2::cD or A3D1D2D3 have a pH of approximately 6.2. To examine the influence of a pH, the pH of the cultures was adjusted either by the additional of HCl or NaOH in order to obtain the required pH.

*Plasmid constructions*. The plasmid that expresses the MSA2::cD fusion has been described in example 1. Plasmid pPA43 is based on the same expression plasmid and contains an in frame fusion of the lactococcal signal sequence of Usp45 (ssUsp; van Asseldonk et al. 1990. Gene 95: 155- 160), the c-myc epitope for detection purposes, the A3 cA repeat and repeats D1, D2 and D3 of cD. Primers that were used for cloning A3 were cArepeat3.fw (CCG TCT CCA ATT **CAA TCT GCT GCT GCT TCA AAT CC)** and cA repeat3.rev (TAA TAA GCT TAA AGG TCT CCA ATT CC**T TTT ATT CGT AGA TAC TGA CCA ATT AAA ATA G)** [in bold are the A3 specific sequences]. The primers used for cloning the three cD repeats were cDrepeat1.fw (CCGTCTCCAATTT**CAGGAGGAACTGCTGTTACAACTAG)** and cDrepeat3.rev (TAATAAGCTTAAA**GGTCTCCAATTCCAGCAACTTGCAAAACTTCTCCTA C)** [in bold are the cD specific sequences].

### Results and Discussion

*Binding of MSA2::cD at low pH.* Since binding of MSA2::cD was not observed at a pH (the pH of the culture medium after growth and induction is about 6.2) higher than the calculated pI for the cD domain (pI 3.85), we studied the binding when the pH of the medium was adjusted to pH3.2. TCA-pretreated *L. lactis* cells were used as the binding substrate and the relative amounts of bound MSA2::cD were analyzed in Western blots. The amounts of unbound reporter protein left behind in the culture supernatant after binding were also analyzed. Fig. 17 shows that there is a clear increase in bound MSA2::cD when binding is performed at pH3.2 (compare lanes 1 and 3). At the same time less unbound reporter protein remained in the supernatant (compare lanes 2 and 4). This result indicates that positive charges are important for binding of cA-type anchoring domains.

*Binding of cAcD hybrid anchors*. Analysis of the pI values of the cA homologs in Table 3 learns that two classes of repeats can be distinguished: a majority (99 out of 148) of homologs that have a high pI value (> 8) and a group (33 out 148), of which cD is a representative, that has pI values lower than 6. Based on our experimental results it is shown that these types of anchoring domains only bind to bacterial cell walls at a pH that is lower than its pI. Notably, most cell wall binding domain homologs consist only of repeats with a pI that are representatives of one of the two groups, i.e. only repeats with a high or low pI. Interestingly, some proteins with cell wall binding domains, e.g. those of DniR of *Trepanoma pallidum* and an amidase of *Borrelia burgdorferi*, consist of repeats with high and low pI. Since the binding pH of such 'natural hybrid' cell-wall binding domains is below the intermediate pI value of the total number of repeats present in the domain, we constructed, using the cA and cD repeats that we have available, a hybrid cell-wall protein anchor that has an intermediate pI value. Table 5 lists both the native AcmA and AcmD anchors a number of examples of cA/cD hybrids. The hybrid protein anchor constructed (A3 D1D2D3) has a calculated pI value of approx. 5.1. A protein anchor consisting of only D1D2D3 shows little binding at a pH above its calculated pI (see above). The A3 (pI 10) domain shows similar binding at pH 5 and pH7.

The binding of the hybrid anchor A3D1D2D3 was tested at pH3, pH 5 and pH7. At pH3 almost all protein had been bound to the ghost cells (Fig. 18). At pH 5 there was still considerable binding (+/- 40%), whereas there was only minimal binding at pH7 (+/- 20%). This result shows that the pH range of binding for cD repeats was shifted to higher pH values by the addition of one cA repeat (A3) that caused a shift in calculated pI values of 3.8 to 5.1. The increase of binding at pH5 for the A3D1D2D3 hybrid can not be attributed to binding of the A3 repeat alone. If this was the case then the same level of binding should occur at pH7 since the A repeats show the same binding at these pH values. In addition, the increased binding at pH5 is not an additive effect in the sense that an extra binding domain results in increased binding. It was shown before that addition of one repeat to the cA anchor did not result in increased binding. The binding at higher pH values of the A3D1D2D3 repeats as compared to D1D2D3 repeats alone has therefore to be attributed to the increase in the calculated pI value of the hybrid cA/cD anchor.

This clearly demonstrates that pH binding properties of these types of protein anchors can be manipulated on the basis of the pI values of individual repeats present in the hybrid anchor.

### EXAMPLE 4

### Induction of cellular immune responses in mice after oral immunizations with lactococcal ghosts displaying the malaria Plasmodium falciparum antigen MSA2 fused to the lactococcal AcmA protein anchor

Non-genetically modified non-living *Lactococcus lactis* cells (ghosts) preloaded with the *Plasmodium falciparum* MSA2 antigen fused to the AcmA protein anchor (MSA2::cA) were used to orally immunize mice in a similar way as described in example 2. In this experiment we specifically addressed the question whether immunizations through the oral route with the non-recombinant non-living Ghosts carrying MSA2::cA on their surface (Ghosts-MSA2::cA) can elicit typical Th1-type immune responses, such as IgG2 antibodies and gamma-interferon (γIFN) producing T cells in the spleen. These types of responses are particularly relevant to obtain immunity for pathogens, like malaria, that undergo stages in their life cycle where they are not in the blood but hide in cells.

### Materials and Methods

Groups of five mice of different strains were used for immunization. The strains used in were Balb/c [with the major histocompatibility locus allotype of H2d], C57 Black [H2b], C3H [H2k] and ICR [out bred, i.e. of varying H2 types]. Oral immunizations were performed at three weekly intervals. Immunizations were performed with MSA2::cA absorbed on to the surfaces of TCA treated *Lactococcus lactis* cells (Ghosts-MSA2::cA) or with recombinant *L*. *lactis* that displayed MSA2 on the surface through the use of a covalently linked cell wall anchor (*L*. *lactis*[MSA2::cP]) as described in example 2. The mice were tail bled to obtain serum samples, two weeks after the second, third and fourth immunizations. Faecal pellets were collected and extracted to examine intestinal IgA antibody production. The mice were sacrificed at the end of each experiment and the spleens were removed for examining T cell responses by ELISPOT. MSA2-his tag produced in *E*. *coli* was used as antigen in the ELISA and ELISPOT assay.

Growth of bacterial strains, preparation of Ghost cells was as described in example 2.

### Results and Discussion

### Kinetics and isotypes of the serum IgG antibodies generated oral immunizations.

Differences in the kinetics of the antibody response and the isotype distribution were observed between different murine strains. Importantly the antibody response was also different when living recombinant *L*. *lactis*[MSA2::cP] or Ghosts-MSA2::cA were used as immunogens. With Ghosts-MSA2::cA, high serum antibody levels were detectable in the C3H mice after two immunizations. IgG antibodies were detectable in all four murine strains after three and four immunizations. Antibody titres were highest in C3H mice. IgG antibodies that reacted with native MSA2 on parasites were detected in the sera of immune mice by fluorescence microscopy (IFA), confirming that the immunizing form of the protein elicits biologically relevant antibodies. Control immunizations were performed with Ghosts alone. No MSA2 specific antibodies were elicited in this case. In parallel experiments with MSA2cP as immunogen, high serum IgG antibody levels were seen only with Balb/c mice after two immunizations. After three and four immunizations, good antibody responses also developed in C3H mice. Antibody titers were highest in Balb/c mice.
There were significant differences between the strains in the isotypes of the elicited serum IgG antibodies in response to immunization with Ghosts-MSA2::cA. Balb/c mice showed higher levels of IgG2a and IgG2b antibodies, some IgG3 antibodies and negligible IgG1. This demonstrates a possible Th1 bias. On the other hand, C57 Black and C3H mice had high IgG1, IgG2a and IgG2b and lower IgG3 antibodies to MSA2. This is more characteristic of a mixed Th1 and Th2 response. ICR mice, as might have been expected, showed a range of responses. Some ICR mice had the Balb/c and others the C3H/C57Black pattern of IgG isotypes.

*Formation of mucosal antibodies*. IgA antibodies were detected by ELISA in the faecal pellets of the ICR and Balb/c mice but not C3H or C57Black mice, when immunization was performed with either living recombinant *L*. *lactis*(MSA2::cP) or Ghost-MSA2::cA.

*T-cell responses*. The increase of the intensity of the IgG ELISA reactions seen in the mice immunized with Ghosts-MSA2::cA with each immunization demonstrates that boosting is taking place and that there is a Th-dependent antibody response in these animals. The IgG isotype distribution further confirms this conclusion. Therefore, Th cells are generated in ICR, Balb/c, C57 Black and C3H mice.
The ELISPOT assay for detecting gamma-interferon (γIFN) producing cells detects mainly CD8⁺ Tc cells. Such cells are an important component of the immune response to many pathogens, including malaria parasites. His-tagged MSA2 produced in *E*. *coli* was used as antigen in the assay. MSA2-specific γIFN producing cells could be demonstrated in the spleens of Balb/c, C57 Black and C3H that were immunized with Ghosts-MSA2::cA. MSA2-specific γIFN producing cells were not observed in the spleens of control mice immunized with Ghosts alone or with the living recombinant *L*. *lactis*(MSA2-cP). The latter group showed a high level of non-specific γIFN producing cells. The reason for the high background may be ongoing inflammation.
The sensitization of MSA2-specific Tc cells in the spleen after immunization with the non-recombinant non-living *L. lactis* Ghost-system that carries a foreign protein is a novel finding. It is applicable to malaria also since protection against sporozoite-infection is associated with γIFN producing cells being produced in the spleen.

In conclusion, the non-recombinant non-living Ghost system can be used in oral immunizations to elicit typical Th1-type immune responses These types of responses are particularly relevant to obtain immunity for pathogens that undergo stages in their life cycle where they are not in the blood but hide in cells. The responses are more pronounced and more specific for the Ghost system than for the living recombinant system. The Ghost system having the additional advantage of eliminating the risk of spreading recombinant DNA into the environment.

### EXAMPLE 5

### Protection of mice for lethal Streptococcus pneumoniae challenge after oral immunizations with lactococcal ghosts preloaded with PpmA antigen fused to the lactococcal AcmA protein anchor

*Streptococcus pneumoniae* is the leading etiological agent of severe infections such as septicemia, meningitis, pneumonia, and otitis media. Recent studies on the molecular epidemiology and pathogenesis of *S*. *pneumoniae*, have identified pneumococcal proteins with vaccine potential. One of these proteins, the protease maturation protein PpmA, has been shown to elicit immune protective potential in a mouse pneumonia model.
The non-genetically modified lactococcal ghosts have been shown to be an efficient carrier in oral immunizations of rabbits and mice to elicit strong anti-malaria immune responses. Here describe the construction of lactococcal ghosts that display on their surface the *S*. *pneumoniae* PpmA fused to the lactococcal AcmA cell-wall binding domain and we investigated the ability of these ghosts to protect orally immunized mice from a lethal nasal dose of *S*. *pneumoniae*.

### Materials and Methods

*Bacterial strains and growth conditions*. *L*. *lactis* was grown and ghost cells were prepared as described in example 1. *S*. *pneumoniae* was grown as described before (Gingles et al. 2001. Infect Immun 69: 426-434).
*Construction ppmA Protein Anchor fusion expression plasmid*. The expression plasmid for ppmA protein anchor fusion(PpmA::cA) was basically similar to the expression plasmid for the MSA2 protein anchor fusion as described in example 2. For the secretion of PpmA::cA use was made of the secretion signal sequence of the Usp45 protein (ssUsp) of *L. lactis* (van Asseldonk et al. 1990. Gene 95: 155- 160). The PpmA gene was cloned by PCR using the primers ppmA.1 (CGGTCTCA*CATG***TCGAAAGGGTCAGAAGGTGCAGACC)** and ppmA.2 (CGGTCTCG*AATT*GC**TTCGTTTGATGTACTACTGCTTGAG)** resulting in plasmid pPA32 that contains ppmA as an in frame fusion with ssUsp45 and the protein anchor (ssUsp::ppmA::cA). Expression of the fusion gene results in the secreted product PpmA::cA. The underlined sequences in the primers indicate an *Eco*31I restriction enzyme recognition site that was for digestion of the PCR fragment. This restriction results in *Nco*I and *Eco*RI sticky ends, which were used for cloning. In bold are the ppmA sequences. Chromosomal DNA of S. *pneumoniae* strain D39 was used as a template.

*Preparation of the vaccine.* Three liters of M17 medium with PpmA::cA obtained after growth and induction for expression of *L*. *lactis*[pPA32] was centrifuged and filter (0.2 µm) sterilized to remove all producer cells. Ghost cells were prepare from 0.5 liter of *L*. *lactis* NZ9000(ΔacmA). After binding the ghost cells with PpmA::cA (Ghosts-PpmA::cA) were isolated by centrifugation and were washed with PBS. Finally they were stored in PBS in aliquots of 2.5 x 10¹⁰ Ghosts/ml at -80°C. Two control groups were included: (i) Ghosts without bound PpmA::cA. For the sample preparation the same amounts of ghost cells were used and the same centrifugation and washing steps were performed, but the binding step was omitted; (ii) soluble PpmA, which was isolated as a his-tagged fusion.

*Mice immunizations*. Groups of 10 mice (CD-1) were used in the immunizations. Oral doses consisted of 5 x 10⁹ Ghosts with or without PpmA::cA (50 µg) or 50 µg soluble PpmA in PBS. Nasal doses contained 5 x 10⁸ Ghosts with or without PpmA::cA (5 µg) or 5 µg soluble PpmA. Subcutaneously, 10⁸ Ghosts-PpmA::cA (1 µg) were injected. For the intranasal immunizations the mice were slightly anesthetized Isofluorane.

*Intranasal challenge*. The groups of orally immunized mice were intranasally challenged 14 days after the last booster immunization with a dose of 10⁶ colony forming units (CFU) *S*. *pneumoniae* D39 as described before (Kadioglu et al. 2000 Infect Immun 68: 492-501). Mice were monitored after the challenge for visible clinical symptoms for 7 days, at which point the experiment was ended. Mice that were alive at this point were considered to have survived the pneumococcal challenge; mice that became moribund during the 7-day period were judged to have reached the endpoint of the assay. The time that the animal became moribund was recorded, and the animal was killed by cervical dislocation.

*ELISA analysis*. Serum samples were taken from each mice before the challenge and stored at -20°C before use. Microtiter plates were coated with 100 µg PpmA/ml in 0.05 carbonate buffer. Serial 10-fold dilutions of pooled serum of each group were incubated on the plates as described before (Gingles et al. 2001. Infect Immun 69: 426-434). Anti-mouse immunoglobulins-horse-radish peroxidase conjugate was used for detection and the absorbance was measured at 492 nm.

### Results and Discussion

*Serum antibody response*. Mice were immunized orally, nasally and subcutaneously according to the scheme shown in Fig. 19. Anti-PpmA antibody titers in the blood serum were determined for each group by ELISA assays. The results are given in Fig. 20. Ghosts alone either orally or nasally administered (OV Ghosts and IN Ghosts) did not induce anti-PpmA antibodies as expected. Soluble PpmA given by the nasal route resulted in only a low anti-PpmA antibody titer, which is in agreement with the general findings that soluble antigens are not very immunogenic when given by the mucosal routes. Ghosts-PpmA::cA provided by the oral route (OV PpmA+Ghost) induced also only a low level of anti-PpmA serum antibodies. This is in contrast to the results for the oral immunization experiments described in examples 2 and 4 with MSA2::cA. However, this may be antigen-type related. Intranasal administration of Ghosts-PpmA::cA resulted in a high titer of anti-PpmA antibodies (IN PpmA+Ghosts). Also high titer were obtain by subcutaneous administration of Ghosts-PpmA::cA. These titers were only a factor 5 to 10 lower compared to soluble PpmA that was subcutaneously administered formulated with the strong Freunds complete adjuvant (Peter Adrian, Erasmus university Rotterdam, The Netherlands, unpublished results). In addition, the Freunds PpmA vaccine contained 50 µg PpmA per dose, whereas the intranasally administered Ghosts-PpmA:cA contains only 5 µg/dose and the subcutaneous Ghost-PpmA::cA vaccine only 1 µg PpmA/dose. This result clearly demonstrates the adjuvant effect of the ghost cells. Side effects of the orally, nasally or subcutaneously administrated ghosts were not observed, this in contrast to the severe side effects that are usually seen with the use of Freunds adjuvants.
The results demonstrate that high titer serum antibodies can be obtained by the mucosal route of adminstration. Also, these data show that ghost cell can be safely used in traditionally injected vaccines without side effects to induce high titer serum antibodies.

*Protection against challenge*. The orally immunized mice with either soluble PpmA, Ghosts alone or Ghosts-PpmA::cA were challenge 14 days post immunizations with a lethal intranasal dose of *S*. *pneumoniae*. The mice immunized with soluble PpmA or Ghosts alone died within 72 hours post challenge. The group immunized with Ghosts-PpmA::cA showed a survival rate of 40% (Fig. 21). This results shows that mucosal immunization of mice with Ghosts-PpmA is able to induce protective immunity against a lethal *S*. *pneumoniae* challenge. In conclusion, the non-recombinant non-living Ghost system can be used to elicit high titer serum antibodies and the mucosal route of administration can be used to obtain protective immunity against an mucosally acquired pathogen.

### References

Bolotin et al. (2001) Genome Res. 11: 731-753.
Buist et al. (1995) J. Bacteriol. 177: 1554-1563.
Gasson (1983) J. Bacteriol. 154: 1-9.
Kok et al. (1988) Appl. Environ. Microbiol. 54: 231-238.
Kuipers et al. (1997) Tibtech. 15: 135-140.
Morata de Ambrosini et al. (1998) J. Food Prot. 61: 557-562.
Navarre and Schneewind (1994) Mol. Microbiol. 14: 115-121.
Norton et al. (1994) FEMS Microbiol. Lett. 120: 249-256.
Norton et al. (1996) FEMS Immunol. Med. Microbiol. 14: 167-177.
Poquet et al. (2000) Mol. Microbiol. 35: 1042-1051.
Ramasamy (1987) Immunol. Cell Biol. 65: 419-424.
Ramasamy et al. (1999) Parasite Immunol. 21: 397-407.
Robinson et al. (1997) Nature Biotechnol. 15: 653-657.
Sauvé et al. (1995) Anal. Biochem. 226: 382-283.

## Claims

1. A method for obtaining spherical peptidoglycan microparticles of a Gram-positive bacterium,
said method comprising treating whole cell material with an acid solution to remove a cell-wall component such as a protein, (lipo)teichoic acid or carbohydrate from said whole cell material to obtain microparticles having an improved capacity as compared to the binding capacity of untreated cell-wall material, for binding with a proteinaceous substance comprising an AcmA cell wall binding domain, or homolog or functional derivative thereof.

2. A method according to claim 1 wherein said substance further comprises a reactive group selected from the group consisting of an antigenic determinant, an enzyme, an antibody or fragment thereof, a polyhistidyl tag, a fluorescing protein, antibiotic, a hormone, a carbohydrate, a fatty acid, an aromatic substance, an inorganic particle and a reporter molecule.

3. A method according to claim 1 or 2 wherein said acid solution comprises an acid selected from the group of acetic acid (HAc), hydrochloric acid (HCl), sulphuric acid (H₂SO₄), trichloroacetic acid (TCA), trifluoroacetic acid (TFA), and monochloroacetic acid (MCA).

4. A method according to claim 3 wherein said solution comprises 0.06 to 1.2 M TCA.

5. A method according to anyone of claims 1 to 4 comprising heating said cell-wall material in said solution.

6. A method according to anyone of claims 1 to 5 comprising pelleting said cell-wall material from said solution.

7. A method according to claim any one of claims 1-6, wherein said cell-wall material is derived from a *Lactococcus*, a *Lactobacillus*, a *Bacillus* or a *Mycobacterium spp*.

8. Spherical peptidoglycan microparticle of a Gram-positive bacterium obtainable by a method according to any one of claims 1 to 7, said microparticle reflecting the size and shape of the bacterium from which it is obtained.

9. Microparticle according to claim 8 provided with a proteinaceous substance comprising an AcmA cell wall binding domain or homolog or functional derivative thereof.

10. Microparticle according to claim 9 wherein said substance further comprises a reactive group selected from the group consisting of an antigenic determinant, an enzyme, an antibody or fragment thereof, a polyhistidyl tag, a fluorescing protein, an antibiotic, a hormone, a carbohydrate, a fatty acid, an aromatic substance, an inorganic particle and a reporter molecule.

11. Use of a microparticle according to any one of claims 8 to 10 for the preparation of a pharmaceutical composition.

12. Use according to claim 11 wherein said composition comprises a vaccine.

13. Use according to claim 12 wherein said vaccine is useful for mucosal immunisation.

14. A pharmaceutical composition comprising a spherical peptidoglycan microparticle according to any one of claims 8 to 10.

15. Use of a spherical peptidoglycan microparticle according to any one of claims 8 to 10 for the preparation of a biocatalyst.

16. A method for binding of a proteinaceous substance to cell-wall material of a Gram-positive bacterium, said substance comprising an AcmA cell wall binding domain or homologue or functional derivative thereof, said method comprising obtaining spherical peptidoglycan microparticles according to a method of any one of claims 1-7, and contacting said substance with said spherical peptidoglycan microparticles.

17. A method according to claim 16 wherein said substance is contacted with said spherical peptidoglycan microparticles at a pH that is lower than the calculated pI value of said AcmA cell wall binding domain or homologue or functional derivative thereof.

## Patentansprüche

1. Verfahren zur Gewinnung von sphärischen Peptidoglycan-Mikroteilchen eines Gram-positiven Bakteriums, wobei das Verfahren das Behandeln von intaktem Zellmaterial mit einer Säurelösung umfasst, so dass eine Zellwandkomponente, wie ein Protein, (Lipo)teichonsäure oder ein Kohlenhydrat, aus dem intakten Zellmaterial entfernt wird, wobei man Mikroteilchen mit einer verbesserten Kapazität im Vergleich zur Bindungskapazität von unbehandeltem Zellwandmaterial erhält, zum Binden an eine proteinartige Substanz, die eine AcmA-Zellwandbindungsdomäne oder ein Homologes oder funktionelles Derivat davon enthält.

2. Verfahren gemäß Anspruch 1, wobei die Substanz weiterhin eine reaktive Gruppe umfasst, die aus der Gruppe ausgewählt ist, die aus einer antigenen Determinante, einem Enzym, einem Antikörper oder Fragment davon, einer Polyhistidyl-Markierung, einem fluoreszierenden Protein, Antibiotikum, Hormon, Kohlenhydrat, einer Fettsäure, einer aromatischen Substanz, einem anorganischen Teilchen und einem Reportermolekül besteht.

3. Verfahren gemäß Anspruch 1 oder 2, wobei die Säurelösung eine Säure umfasst, die aus der Gruppe Essigsäure (HAc), Chlorwasserstoffsäure (HCl), Schwefelsäure (H₂SO₄), Trichloressigsäure (TCA), Trifluoressigsäure (TFA) und Monochloressigsäure (MCA) ausgewählt ist.

4. Verfahren gemäß Anspruch 3, wobei die Lösung 0,06 bis 1,2 M TCA umfasst.

5. Verfahren gemäß einem der Ansprüche 1 bis 4, das das Erhitzen des Zellwandmaterials in der Lösung umfasst.

6. Verfahren gemäß einem der Ansprüche 1 bis 5, das das Abzentrifugieren des Zellwandmaterials aus der Lösung umfasst.

7. Verfahren gemäß einem der Ansprüche 1 bis 6, wobei das Zellwandmaterial von einer *Lactococcus-, Lactobacillus-, Bacillus-* oder *Mycobacterium-*Spezies stammt.

8. Sphärisches Peptidoglycan-Mikroteilchen eines Gram-positiven Bakteriums, das nach einem Verfahren gemäß einem der Ansprüche 1 bis 7 erhältlich ist, wobei das Mikroteilchen die Größe und Form des Bakteriums, von dem es erhalten wurde, widerspiegelt.

9. Mikroteilchen gemäß Anspruch 8, das mit einer proteinartigen Substanz, die eine AcmA-Zellwandbindungsdomäne oder ein Homologes oder funktionelles Derivat davon umfasst, versehen ist.

10. Mikroteilchen gemäß Anspruch 9, wobei die Substanz weiterhin eine reaktive Gruppe umfasst, die aus der Gruppe ausgewählt ist, die aus einer antigenen Determinante, einem Enzym, einem Antikörper oder Fragment davon, einer Polyhistidyl-Markierung, einem fluoreszierenden Protein, Antibiotikum, Hormon, Kohlenhydrat, einer Fettsäure, einer aromatischen Substanz, einem anorganischen Teilchen und einem Reportermolekül besteht.

11. Verwendung eines Mikroteilchens gemäß einem der Ansprüche 8 bis 10 zur Herstellung einer pharmazeutischen Zusammensetzung.

12. Verwendung gemäß Anspruch 11, wobei die Zusammensetzung einen Impfstoff umfasst.

13. Verwendung gemäß Anspruch 12, wobei der Impfstoff für die mukosale Immunisierung geeignet ist.

14. Pharmazeutische Zusammensetzung, die ein sphärisches Peptidoglycan-Mikroteilchen gemäß einem der Ansprüche 8 bis 10 umfasst.

15. Verwendung eines sphärischen Peptidoglycan-Mikroteilchens gemäß einem der Ansprüche 8 bis 10 zur Herstellung eines Biokatalysators.

16. Verfahren zur Bindung einer proteinartigen Substanz an Zellwandmaterial eines Gram-positiven Bakteriums, wobei die Substanz eine AcmA-Zellwandbindungsdomäne oder ein Homologes oder funktionelles Derivat davon umfasst, wobei das Verfahren das Gewinnen von sphärischen Peptidoglycan-Mikroteilchen nach einem Verfahren gemäß einem der Ansprüche 1 bis 7 und das In-Kontakt-Bringen der Substanz mit den sphärischen Peptidoglycan-Mikroteilchen umfasst.

17. Verfahren gemäß Anspruch 16, wobei die Substanz bei einem pH-Wert, der kleiner ist als der berechnete pI-Wert der AcmA-Zellwandbindungsdomäne oder des Homologen oder funktionellen Derivats davon, mit den sphärischen Peptidoglycan-Mikroteilchen in Kontakt gebracht wird.

## Revendications

1. Procédé d'obtention de microparticules de peptidoglycane sphériques d'une bactérie Gram positive,
ledit procédé comprenant le traitement de l'ensemble du matériau cellulaire avec une solution acide pour éliminer un composant de paroi cellulaire tel qu'une protéine, l'acide (lipo)téichoïque ou un glucide dudit ensemble du matériau cellulaire pour obtenir des microparticules ayant une capacité améliorée par rapport à la capacité de liaison du matériau de paroi cellulaire non traité, pour liaison avec une substance protéique comprenant un domaine de liaison de paroi cellulaire AcmA, ou un homologue ou un dérivé fonctionnel de celui-ci.

2. Procédé selon la revendication 1, dans lequel ladite substance comprend en outre un groupe réactif choisi dans le groupe constitué d'un déterminant antigénique, d'une enzyme, d'un anticorps ou d'un fragment de celui-ci, d'un marqueur poly(histidyle), d'une protéine fluorescente, d'un antibiotique, d'une hormone, d'un glucide, d'un acide gras, d'une substance aromatique, d'une particule inorganique et d'une molécule rapportrice.

3. Procédé selon la revendication 1 ou 2, dans lequel ladite solution acide comprend un acide choisi dans le groupe constitué de l'acide acétique (HAc), de l'acide chlorhydrique (HCl), de l'acide sulfurique (H2ₛO₄), de l'acide trichloroacétique (TCA), de l'acide trifluoroacétique (TFA) et de l'acide monochloroacétique (MCA).

4. Procédé selon la revendication 3, dans lequel ladite solution comprend 0,06 à 1,2 M de TCA.

5. Procédé selon l'une quelconque des revendications 1 à 4, comprenant le chauffage dudit matériau de paroi cellulaire dans ladite solution.

6. Procédé selon l'une quelconque des revendications 1 à 5, comprenant la formation en pastille dudit matériau de paroi cellulaire de ladite solution.

7. Procédé selon l'une quelconque des revendications 1 à 6, dans lequel ledit matériau de paroi cellulaire est dérivé d'un *Lactococcus*, d'un *Lactobacillus*, d'un *Bacillus* ou d'un *Mycobacterium spp.*

8. Microparticule de peptidoglycane sphérique d'une bactérie Gram positive pouvant être obtenue par un procédé selon l'une quelconque des revendications 1 à 7, ladite microparticule reflétant la taille et la forme de la bactérie à partir de laquelle elle est obtenue.

9. Microparticule selon la revendication 8, dotée d'une substance protéique comprenant un domaine de liaison de paroi cellulaire AcmA ou un homologue ou un dérivé fonctionnel de celui-ci.

10. Microparticule selon la revendication 9, dans laquelle ladite substance comprend en outre un groupe réactif choisi dans le groupe constitué d'un déterminant antigénique, d'une enzyme, d'un anticorps ou d'un fragment de celui-ci, d'un marqueur poly(histidyle), d'une protéine fluorescente, d'un antibiotique, d'une hormone, d'un glucide, d'un acide gras, d'une substance aromatique, d'une particule inorganique et d'une molécule rapportrice.

11. Utilisation d'une microparticule selon l'une quelconque des revendications 8 à 10 pour la préparation d'une composition pharmaceutique.

12. Utilisation selon la revendication 11, dans laquelle ladite composition comprend un vaccin.

13. Utilisation selon la revendication 12, dans laquelle ledit vaccin est utile pour l'immunisation mucosale.

14. Composition pharmaceutique comprenant une microparticule de peptidoglycane sphérique selon l'une quelconque des revendications 8 à 10.

15. Utilisation d'une microparticule de peptidoglycane sphérique selon l'une quelconque des revendications 8 à 10 pour la préparation d'un biocatalyseur.

16. Procédé de liaison d'une substance protéique au matériau de paroi cellulaire d'une bactérie Gram positive, ladite substance comprenant un domaine de liaison de paroi cellulaire AcmA ou un homologue ou un dérivé fonctionnel de celui-ci, ledit procédé comprenant l'obtention de microparticules de peptidoglycane sphériques selon un procédé conforme à l'une quelconque des revendications 1 à 7, et la mise en contact de ladite substance avec lesdites microparticules de peptidoglycane sphériques.

17. Procédé selon la revendication 16, dans lequel ladite substance est mise en contact avec lesdites microparticules de peptidoglycane sphériques à un pH qui est inférieur à la valeur pI calculée dudit domaine de liaison de paroi cellulaire AcmA ou homologue ou dérivé fonctionnel de celui-ci.
